(19) Europäisches Patentamt
European Patent Office
Office européen des brevets

(11) **EP 3 288 896 B1**

(12) **EUROPÄISCHE PATENTSCHRIFT**

(45) Veröffentlichungstag und Bekanntmachung des Hinweises auf die Patenterteilung:
**19.09.2018 Patentblatt 2018/38**

(21) Anmeldenummer: **16718644.4**

(22) Anmeldetag: **21.04.2016**

(51) Int Cl.:
*C01B 33/152* *(2006.01)*  *C01B 33/155* *(2006.01)*
*C01B 33/158* *(2006.01)*

(86) Internationale Anmeldenummer:
**PCT/EP2016/058834**

(87) Internationale Veröffentlichungsnummer:
**WO 2016/173912 (03.11.2016 Gazette 2016/44)**

(54) **HYDROPHOBE AEROGELE MIT EINER GERINGEN BELEGUNG AN MONOFUNKTIONELLEN EINHEITEN**

HYDROPHOBIC AEROGELS COMPRISING LOW OCCUPANCY OF MONOFUNCTIONAL UNITS

AÉROGELS HYDROPHOBES AYANT UNE TENEUR RÉDUITE EN MOTIFS MONOFONCTIONNELS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.04.2015 DE 102015207945**

(43) Veröffentlichungstag der Anmeldung:
**07.03.2018 Patentblatt 2018/10**

(73) Patentinhaber: **Wacker Chemie AG**
**81737 München (DE)**

(72) Erfinder:
• **HINDELANG, Konrad**
**80939 München (DE)**
• **JANTKE, Dominik**
**85386 Eching (DE)**
• **WEIDNER, Richard**
**84489 Burghausen (DE)**

(74) Vertreter: **Fritz, Helmut et al**
**Wacker Chemie AG**
**Zentralbereich Patente, Marken und Lizenzen**
**Hanns-Seidel-Platz 4**
**81737 München (DE)**

(56) Entgegenhaltungen:
**DE-A1- 19 648 798    US-A- 4 954 327**
**US-A- 5 789 495**

**Beschreibung**

Gegenstand der Erfindung sind Gele ausgewählt aus Lyo- oder Aerogelen enthaltend Primärpartikel, die aus oxidischen Einheiten und $[R_xSiO_{(4-x)/2}]$-Einheiten aufgebaut sind, wobei die Primärpartikel eine Änderung der Konzentration an $[R_xSiO_{(4-x)/2}]$-Einheiten von innen nach außen aufweisen, wobei x gleich oder verschieden sein kann und 1 oder 2 ist und R gleich oder verschieden sein kann und Wasserstoff oder ein organischer, substituierter oder unsubstituierter Rest ist und wobei die oxidischen Einheiten $[SiO_{4/2}]$-Einheiten enthalten, sowie ein Verfahren zu ihrer Herstellung. Ein weiterer Gegenstand der Erfindung ist die Verwendung der erfindungsgemäßen Gele in kosmetischen oder medizinischen Anwendungen, als Katalysator oder Katalysatorträger, für chromatographische Anwendungen, oder, wenn es sich bei den Gelen um Aerogele handelt, zur thermischen und/oder akustischen Isolierung.

Die Wärmedämmung zur Einsparung von Energie hat im Rahmen des Bewusstwerdens für nachhaltige Entwicklung und der Verteuerung der Energie sowie sich verknappenden fossilen Rohstoffen einen hohen Stellenwert erhalten. Diese Anforderungen an eine Optimierung des Wärmedämmschutzes gelten im gleichen Maße sowohl für Gebäude, also für Neubauten oder Bauten im Bestand, als auch für thermische Isolierungen im logistischen oder stationären Bereich.

Vor dem Hintergrund einer nachhaltigen Isolierung, welche eine geringe Wärmeleitung sowie geringe Brennbarkeit aufweist, sind anorganische, poröse Materialien zunehmend im Fokus.

Aerogele mit hohen Porositäten (> 60%) und geringer Dichte (< 0,6 g/ml) weisen eine geringe thermische Leitfähigkeit auf und finden deshalb als thermische Isolatoren ein breites Anwendungsspektrum (M. A. Aegerter et al. (Eds.), Aerogels Handbook Series: Advances in Sol-Gel Derived Materials and Technologies, 1st ed. 2011, Springer Verlag, New York Dordrecht Heidelberg London).

Aufgebaut sind Gele, insbesondere $SiO_2$-Gele aus Netzwerken, welche sich aus Primärpartikeln zusammensetzen, die nach ihrer Verknüpfung und Versinterung der Kontaktflächen in einem Sol-Gel-Prozess, stabile, flüssigkeitsgefüllte Netzwerke, sogenannte Lyogele, bilden. Diese Lyogele können durch Entfernen des Lösungsmittels in Aerogele überführt werden. Die Poren des Aerogels sind dementsprechend mit Luft gefüllt. Während bei einem Lyogel die Poren mit Lösungsmittel gefüllt sind, stellt ein Hydrogel einen Spezialfall des Lyogels dar, bei dem die Porenflüssigkeit zu mindestens 50% aus Wasser besteht.

Es ist wünschenswert, eine möglichst hohe Hydrophobie der $SiO_2$-Aerogele zu erreichen, um die Wasseraufnahme und dadurch den Verlust der thermischen Isolierungswirkung zu reduzieren. Erreicht wird eine permanente Hydrophobie durch Behandlung der Oberfläche von Gelnetzwerken mit hydrophoben Gruppen, bevorzugt durch Modifizierung.

Zudem ist es wünschenswert, Aerogele mit möglichst geringer Brennbarkeit und daher einem möglichst geringen Kohlenstoffgehalt (C-Gehalt) herzustellen.

Für weiterführende Anwendungen in der thermischen Isolierung ist zudem eine möglichst hohe Stabilität gegenüber mechanischen Einflüssen und Flexibilität der zu verwendenden Wärmeisolationsmaterialien wünschenswert.

Anorganische Aerogele, speziell basierend auf $SiO_2$, sind bereits seit 1931 bekannt (s. z.B. Aegerter et al., Aerogels Handbook 2011, s.o.). Diese werden durch den Aufbau eines Netzwerkes aus $SiO_2$-Primärpartikeln über einen Sol-Gel-Prozess hergestellt.

In EP 0 948 395 B1 ist die Herstellung von hydrophoben Aerogelen auf Basis von $SiO_2$ beschrieben. Hierbei erfolgt die Netzwerkbildung in wässrigen Lösungen zu Hydrogelen, welche anschließend durch Silylierung der an der Oberfläche zugänglichen Si-OH Gruppen modifiziert werden und dann unterkritisch zu Aerogelen getrocknet werden. Die nachträgliche Silylierung führt zu einem Belegungsgrad mit Trimethylsilyl-Gruppen (TMS, $(CH_3)_3Si\_$) von mindestens 2,6 $nm^{-2}$, der C-Gehalt liegt dementsprechend in keinem Fall unter 6,8 Gew.-% (s. Tabelle 1). Zudem haben diese Netzwerke die für Aerogele allgemein im Stand der Technik bekannten Nachteile, dass sie brüchig und spröde sind (s. beispielsweise Einleitung A. V. Rao et al., J. Colloid Interface Sci. 300, 2006, p. 279-285; oder Aegerter et al. Aerogels Handbook 2011, s.o.), da sie aus starren $SiO_2$-Gerüsten bestehen.

Im Stand der Technik bekannt ist ebenso die Belegung der Oberfläche von fertig aufgebauten $SiO_2$-Gelnetzwerken (als Hydro- oder Lyogel) mit anderen Silanen, wodurch das starre $SiO_2$-Gerüst mit einer Schicht aus $[R_xSiO_{(4-x)/2}]$ (mit x=1 oder 2) belegt wird.

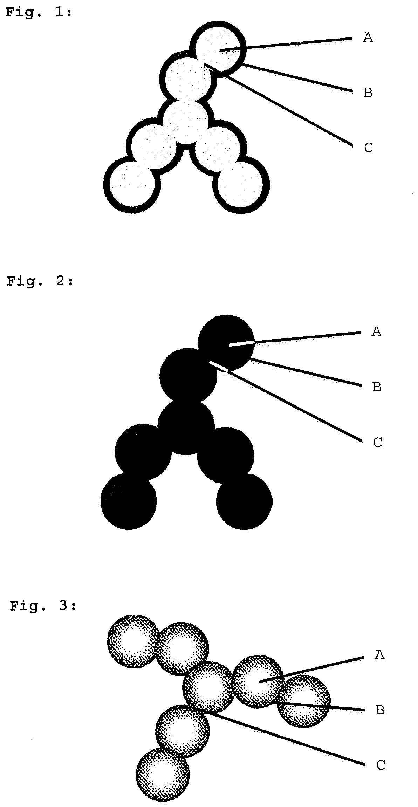

Dieser Aufbau hat jedoch den Nachteil, dass eine nachträgliche Modifizierung des Gelnetzwerkes mit Alkoxysilanen zur Netzwerkverstärkung mit mehrstufigen und damit aufwändigen Lösungsmittelaustauschschritten verbunden ist (A. V. Rao et al., Appl. Surf. Sci. 253, 2007, p. 6032-6040). Da die Primärpartikel direkt verbunden sind, d.h. die Kontaktstellen zwischen den Primärpartikeln genau wie in EP 0 948 395 beschrieben auch in diesem Fall herstellungsbedingt über starre $SiO_2$-Brücken zustande kommen, liefert dieses Verfahren ebenso spröde Produkte (s. Fig. 1). Da die interpartikulären Verknüpfungen bereits vor der Modifizierung ausgebildet wurden, werden diese aus $[SiO_{4/2}]$-Einheiten gebildet (in Fig. 1 hell dargestellt).

Ein alternativer Ansatz, nämlich der direkte Aufbau von Aerogelen aus $[CH_3SiO_{3/2}]$-Einheiten ohne nachträgliche Silylierung, wurde in den Publikationen von A. V. Rao et al. (2006, s.o.) und K. Kanamori et al. (Adv. Mater. 19, 2007, p. 1589-1593) beschrieben. Als Produkte wurden hochflexible Aerogele erhalten. Diese wiesen allerdings trotz des Verzichts auf eine nachträgliche Silylierung durch den hohen Kohlenstoffanteil im verwendeten Ausgangsstoff einen hohen C-Gehalt auf, der mit 17,9 Gew.-% berechnet wurde. Die Brennbarkeit dieser Gele ist also im Vergleich zu den Produkten aus EP 0 948 395 erhöht (s. Fig. 2). Das gesamte Gelnetzwerk und damit auch die interpartikulären Verknüpfungen bestehen aus $[R_xSiO_{(4-x)/2}]$-Einheiten (in Fig. 2 schwarz dargestellt). Abhängig von den eingesetzten Ausgangsstoffen weist das Gel daher einen sehr hohen C-Gehalt auf.

Es stellte sich daher die Aufgabe, Aerogele mit möglichst hoher und permanenter Hydrophobie zu erzeugen, die eine reduzierte Brennbarkeit, also einen möglichst geringen Kohlenstoffgehalt aufweisen und gleichzeitig weniger starr und spröde sind, als bekannte Systeme, d.h. die bei reduzierter Brennbarkeit eine hohe Flexibilität besitzen.

Gelöst wird diese Aufgabe durch die Erfindung, die Gele ausgewählt aus Lyo- oder Aerogelen enthaltend Primärpartikel, die aus oxidischen Einheiten und $[R_xSiO_{(4-x)/2}]$-Einheiten aufgebaut sind, wobei die Primärpartikel eine Änderung der Konzentration an $[R_xSiO_{(4-x)/2}]$-Einheiten von innen nach außen aufweisen, wobei x gleich oder verschieden sein kann und 1 oder 2 ist und R gleich oder verschieden sein kann und Wasserstoff oder ein organischer, substituierter oder unsubstituierter Rest ist und wobei die oxidischen Einheiten $[SiO_{4/2}]$-Einheiten enthalten, zur Verfügung stellt.

Bevorzugt enthaltenen die Gele ausgewählt aus Lyo- oder Aerogelen, nur Primärpartikel, die aus oxidischen Einheiten und $[R_xSiO_{(4-x)/2}]$-Einheiten bestehen, wobei die Primärpartikel eine Änderung der Konzentration an $[R_x-SiO_{(4-x)/2}]$-Einheiten von innen nach außen aufweisen, wobei x gleich oder verschieden sein kann und 1 oder 2 ist und R gleich oder verschieden sein kann und Wasserstoff oder ein organischer, substituierter oder unsubstituierter Rest ist und wobei die oxidischen Einheiten $[SiO_{4/2}]$-Einheiten enthalten.

Ein Gel im Sinne der vorliegenden Erfindung ist ein disperses System, das aus mindestens zwei Komponenten besteht. Die feste Komponente bildet dabei ein schwammartiges, dreidimensionales Netzwerk, dessen Poren durch eine Flüssigkeit oder ein Gas ausgefüllt sind. Die flüssige oder gasförmige Komponente ist dadurch in der festen immobilisiert. Sind die Poren mit Lösungsmittel gefüllt, liegt ein Lyogel vor. Ist das Netzwerk hochporös und Luft das eingelagerte Gas, so wird das Gel auch als Aerogel bezeichnet. Erfindungsgemäß sind die Gele ausgewählt aus Lyo- oder Aerogelen.

Die Oberfläche der Gele, ermittelt mit der BET-Methode entsprechend DIN 9277/66131 und 9277/66132, liegt bevorzugt im Bereich zwischen 300 und 1000 m²/g, besonders bevorzugt zwischen 300 und 900 m²/g, insbesondere bevorzugt zwischen 300 und 800 m²/g.

Als Primärpartikel werden im Sinne der Erfindung Nanopartikel verstanden, welche im Sinne eines Sol-Gel-Prozesses durch Agglomeration und Versinterung an den Kontaktflächen die netzwerkbildenden Einheiten des Gels darstellen.

Unter einem Sol-Gel-Prozess versteht man ein Verfahren zur Herstellung nichtmetallischer anorganischer oder hybridpolymerer Materialien aus kolloidalen Dispersionen, den sogenannten Solen (abgeleitet vom englischen solution).

In der vorliegenden Erfindung bezeichnet ein Sol eine Lösung und/oder kolloidale Dispersion von Molekülen und/oder Partikeln in mindestens einem Lösungsmittel bzw. Dispersionsmedium. Eine Dispersion ist ein heterogenes Gemisch aus mindestens zwei Stoffen, die sich nicht oder kaum ineinander lösen oder chemisch miteinander verbinden. Dabei ist ein oder mehrere Stoffe (disperse Phase) fein verteilt in einem anderen kontinuierlichen Stoff (Dispersionsmedium, Synonym = kontinuierliche Phase). Nach ihrer Teilchengröße bezeichnet man als kolloidal dispers gelöst disperse Phasen mit einer Teilchengröße von typischerweise ca. 1 nm bis 1 pm.

Eine Agglomeration (lat.: agglomerare - *zusammenballen, anhäufen*) ist eine mehr oder weniger verfestigte Anhäufung von kleineren Bestandteilen zu einem größeren Verbund

Unter Versinterung versteht man die Verstärkung der Kontaktflächen zwischen den einzelnen agglomerierten Kontaktflächen durch den Einbau von weiteren monomeren oder oligomeren Einheiten.

Die Primärpartikel der erfindungsgemäßen Gele werden auch als optimierte Primärpartikel bezeichnet. Die aus diesen optimierten Primärpartikeln aufgebauten Gelnetzwerke zeichnen sich dadurch aus, dass sie keine direkten Kontakte von rein oxidischen Partikeln zwischen den Partikeln aufweisen, was zu sehr starren und spröden Produkten führen würde. Dadurch haben die Gele nicht den aus dem Stand der Technik bekannten Nachteil der direkten Verknüpfung von Primärpartikeln über starre, aus reinem $SiO_2$ bestehende Sinterbrücken. Gleichzeitig zeichnen sich die aus den optimierten Primärpartikeln aufgebauten Gelnetzwerke dadurch aus, dass der Belegungsgrad mit monofunktionellen Einheiten und damit C-Gehalt geringer ist. D.h. die erfindungsgemäßen Gele besitzen bei reduzierter Brennbarkeit eine hohe Flexibilität und gleichzeitig hohe Stabilität, also hohe mechanische Belastbarkeit.

Erfindungsgemäß enthalten die Gele Primärpartikel, die aus oxidischen Einheiten und $[R_xSiO_{(4-x)/2}]$-Einheiten aufgebaut sind, wobei die oxidischen Einheiten $[SiO_{4/2}]$-Einheiten enthalten, und wobei eine Änderung der Konzentration an $[R_xSiO_{(4-x)/2}]$-Einheiten im Primärpartikel von innen nach außen vorliegt. Bevorzugt sind mindestens 75 %, besonders bevorzugt mindestens 90 % und insbesondere bevorzugt mindestens 99 % der Primärpartikel, die das erfindungsgemäße Gel enthält, optimierte Primärpartikel. In einer besonders bevorzugten Ausführungsform sind alle Primärpartikel, die das erfindungsgemäße Gel enthält, optimierte Primärpartikel. In einer bevorzugten Ausführungsform durchläuft die Konzentration an $[R_xSiO_{(4-x)/2}]$-Einheiten in den optimierten Primärpartikeln von innen nach außen ein Minimum. Besonders bevorzugt weisen die Primärpartikel eine Zunahme der Konzentration an $[R_xSiO_{(4-x)/2}]$-Einheiten von innen nach außen auf.

Durch Bestimmung der elementaren Zusammensetzung eines Gels mittels Elementaranalyse kann die Anwesenheit der Elemente bestätigt werden.

Mittels Kernresonanzspektroskopie kann am Festkörper, das heißt an den trockenen Gelen, das Vorliegen von verschiedenen $[R_xSiO_{(4-x)/2}]$-Einheiten überprüft, sowie eine Abschätzung der Mengenverhältnisse vorgenommen werden. Hierbei kann mittels $^{13}$C NMR Spektroskopie der Substituent R identifiziert werden und mittels $^{29}$Si NMR Spektroskopie aufgrund der unterschiedlichen Verschiebungsbereiche die Anzahl der Substituenten x in den $[R_xSiO_{(4-x)/2}]$-Einheiten mit x = 0 - 4 bestimmt werden. Der Kohlenstoffgehalt kann mit einem Kohlenstoffanalysator beispielsweise der Firma Leco bestimmt werden. Aus der Bestimmung der BET-Oberfläche entsprechend DIN 9277/66131 und 9277/66132 kann in Kombination mit den Ergebnissen der Kernresonanzspektroskopie die Belegung der Oberfläche mit TMS-Gruppen berechnet werden, welche durch den Kohlenstoffgehalt bestätigt wird.

Der berechnete TMS-Gruppen Belegungsgrad kann mit dem hier anhängigen Beispiel zur Cokondensation, also ohne Änderung der Konzentration an $[R_xSiO_{(4-x/2)}]$-Einheiten von innen nach außen, sowie den Beispielen mit einer Zunahme der Konzentration an $[R_xSiO_{(4-x/2)}]$-Einheiten von innen nach außen verglichen werden, und so auf das Vorliegen einer Konzentrationsänderung geschlossen werden. Eine Konzentrationsänderung der $[R_xSiO_{(4-x/2)}]$-Einheiten von innen nach außen liegt vor, wenn der Belegungsgrad mit TMS-Gruppen niedriger als bei einer Cokondensation ist. Idealerweise liegt der Belegungsgrad nach dem erfindungsgemäßen Aufbau bei unter 1,5 $nm^{-2}$.

Der erfindungsgemäße Aufbau der Primärpartikel mit einer Konzentrationsänderung an $[R_xSiO_{(4-x)/2}]$-Einheiten im Primärpartikel kann beispielsweise durch TOF-SIMS (Flugzeit-Sekundärionenmassenspektrometrie), bei welcher unterschiedliche Intensitäten der beobachteten Ionenspur bei zunehmenden Abtrag der Primärpartikel beobachtet werden können, oder durch das Vorliegen unterschiedlicher Kontraste im Zentrum und am äußeren Rand der Primärpartikel durch hochauflösende TEM-Methoden (Transmissionselektronenmikroskopie) nachgewiesen werden.

Im Inneren enthalten die optimierten Primärpartikel also eine Mischung von $[SiO_{4/2}]$-Einheiten mit anderen oxidischen Einheiten, bevorzugt nur $[SiO_{4/2}]$-Einheiten. Da sich erfindungsgemäß die Konzentration an $[R_xSiO_{(4-x)/2}]$-Einheiten (wobei x gleich oder verschieden sein kann und 1 oder 2 ist und R gleich oder verschieden und R gleich Wasserstoff oder ein organischer, substituierter oder unsubstituierter Rest) ändert, besonders bevorzugt von innen nach außen zunimmt, können im Inneren der Primärpartikel in dieser Ausführungsform entweder gar keine $[R_xSiO_{(4-x)/2}]$-Einheiten, oder eine Mischung aus oxidischen Einheiten und $[R_xSiO_{(4-x)/2}]$-Einheiten vorliegen.

Da sich erfindungsgemäß die Konzentration an $[R_xSiO_{(4-x)/2}]$-Einheiten (wobei x gleich oder verschieden sein kann und 1 oder 2 ist und R gleich oder verschieden und R gleich Wasserstoff oder ein organischer, substituierter oder unsubstituierter Rest ist) ändert, besonders bevorzugt von innen nach außen zunimmt, kann in dem besonders bevorzugten Aufbau die Schale beziehungsweise äußere Hülle der Primärpartikel nur $[R_xSiO_{(4-x)/2}]$-Einheiten oder eine Mischung aus oxidischen und $[R_xSiO_{(4-x)/2}]$-Einheiten enthalten.

In einer besonders bevorzugten Ausführungsform liegt die Konzentrationszunahme an $[R_xSiO_{(4-x)/2}]$-Einheiten in den Primärpartikeln von innen nach außen in Form einer graduellen, d.h. allmählichen, nicht abrupten Zunahme, d.h. in Form eines Gradienten vor. Dieser Aufbau wird als Gradientenaufbau bezeichnet (s. Fig.3).

Der molare Anteil an $[R_xSiO_{(4-x)/2}]$-Einheiten im Primärpartikel liegt innen bevorzugt unter 20 mol-%, besonders bevorzugt unter 10 mol-% und insbesondere bevorzugt bei 0 mol-% und wird nach außen bevorzugt auf mehr als 80 mol-% erhöht, besonders bevorzugt auf mehr als 90 mol-% erhöht und insbesondere bevorzugt auf 100 mol-% erhöht.

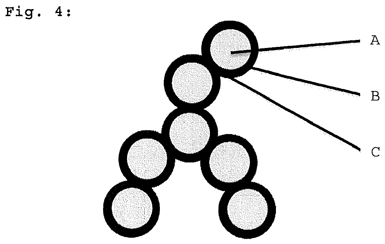

In einer weiteren, besonders bevorzugten Ausführungsform sind die Primärpartikel in Form eines Kern-Schale-Modells aufgebaut, wobei der Kern eine Konzentration an $[R_xSiO_{(4-x)/2}]$-Einheiten von unter 20 mol-% enthält und die Schale eine Konzentration an $[R_xSiO_{(4-x)/2}]$-Einheiten von über 80 mol-% enthält. Der Aufbau der Primärpartikel wird als Kern-Schale-Modell bezeichnet, da die Konzentrationszunahme an $[R_xSiO_{(4-x)/2}]$-Einheiten im Primärpartikel in Form eines sprunghaften Anstiegs vorliegt (zur Verdeutlichung s. Fig. 4). Die Konzentration an $[R_xSiO_{(4-x)/2}]$-Einheiten nimmt in diesem Fall von innen nach außen in Form eines abrupten, scharfen Konzentrationssprungs zu. Ein abrupter, scharfer Konzentrationssprung bedeutet, dass der molare Anteil an $[R_xSiO_{(4-x)/2}]$-Einheiten im Kern bevorzugt unter 20 mol-%, besonders bevorzugt unter 10 mol-% und insbesondere bevorzugt bei 0 mol-% liegt und in einem Konzentrationssprung bevorzugt auf über 80 mol-%, besonders bevorzugt auf über 90 mol-% und insbesondere bevorzugt auf 100 mol-% erhöht wird.

Bevorzugt tragen die optimierten Primärpartikel der Gele außen eine Schicht aus $[R_xSiO_{(4-x)/2}]$-Einheiten, welche für eine komplette Belegung der Partikeloberfläche mit $[R_xSiO_{(4-x)/2}]$-Einheiten ausreicht. Eine komplette Belegung der Partikeloberfläche mit $[R_xSiO_{(4-x)/2}]$-Einheiten bedeutet, dass an der Partikeloberfläche keine $[SiO_{-1/2}]$-Einheiten vorliegen. Die Dicke der Schicht ist dabei so gewählt, dass ein möglichst geringer Anteil an $[R_xSiO_{(4-x)/2}]$-Einheiten aufgebracht wird. Wenn die Primärpartikel entsprechend einem Kern-Schale-Modell aufgebaut sind, ist die Schichtdicke der Schale aus $[R_xSiO_{(4-x)/2}]$-Einheiten bevorzugt kleiner als 3 nm, besonders bevorzugt kleiner als 2 nm und insbesondere bevorzugt kleiner als 1 nm.

Ebenfalls können die erfindungsgemäßen Primärpartikel beide Konzepte vereinen. Somit kann innen eine graduelle Zunahme der Konzentration an $[R_xSiO_{(4-x)/2}]$-Einheiten vorliegen, welche in einem scharfen Konzentrationssprung nach außen erhöht wird.

Ebenfalls können die erfindungsgemäßen Primärpartikel einen Aufbau aufweisen, bei dem die Konzentration an $[R_xSiO_{(4-x)/2}]$-Einheiten im Primärpartikel ein Minimum durchläuft. Hierbei ist es nicht relevant, ob das Minimum durch graduelle Abnahme und anschließende Zunahme oder durch Konzentrationssprünge im Anteil an $[R_xSiO_{(4-x)/2}]$-Einheiten hervorgerufen wird.

Erfindungsgemäß sind die Primärpartikel der Gele aus oxidischen Einheiten und $[R_xSiO_{(4-x)/2}]$-Einheiten aufgebaut, wobei die oxidischen Einheiten $[SiO_{4/2}]$-Einheiten enthalten.
Oxidische Einheiten bezeichnen Verbindungen, in denen ein Metallatom ausschließlich an Sauerstoffatome gebunden ist, die wiederum jeweils ein freies Elektron für eine weitere Bindung aufweisen. Als oxidische Einheiten können $[SiO_{4/2}]$-Einheiten mit allen dem Fachmann bekannten, hydrolysestabilen Metalloxiden oder deren Mischungen vorliegen, bevorzugt liegen zusätzlich zu den $[SiO_{4/2}]$-Einheiten als oxidische Einheiten drei- oder vierwertige Einheiten, besonders bevorzugt nur $[SiO_{4/2}]$-Einheiten vor.

Bei den $[R_xSiO_{(4-x)/2}]$-Einheiten, wobei x gleich oder verschieden sein kann und 1 oder 2 ist, sind neben ein oder zwei Sauerstoffatomen ein oder zwei Reste R direkt an das Siliciumatom gebunden. Es können entweder in allen $[R_xSiO_{(4-x)/2}]$-Einheiten ein Rest R und drei Sauerstoffatome, d.h. x=1, oder in allen $[R_xSiO_{(4-x)/2}]$-Einheiten zwei Reste R und zwei Sauerstoffatome, d.h. x=2, an das Siliciumatom gebunden sein. Es kann auch eine Mischung aus $[RSiO_{3/2}]$-Einheiten und $[R_2SiO_{2/2}]$-Einheiten vorliegen. Auch hier steht $O_{(4-x)/2}$ (z.B. $O_{3/2}$, $O_{2/2}$) für (4-x) (3, 2) Sauerstoffatome, die jeweils ein freies Elektron für eine weitere Bindung aufweisen. Für R gilt die aufgeführte Definition.

Die Reste R können gleich oder verschieden sein und unabhängig voneinander, Wasserstoff, ein organischer, linearer, verzweigter, cyclischer, gesättigter oder ungesättigter, aromatischer oder heteroaromatischer Rest, mit oder ohne Substituenten. Das bedeutet, dass die Reste R substituiert oder unsubstituiert sein können. Bevorzugte Substituenten sind -CN, -NCO, -NR_2, -COOH, -COOR, -Halogen, -(Meth)acryl, -Epoxy, -SH, -OH, -CONR_2, -O-R, -CO-R, -COO-R, -OCO-R, oder -OCOO-R, -S-R, -NR-, -N=R,-N=N-R, oder -P=R. Vorzugsweise werden gesättigte oder ungesättigte Reste mit $C_1$-$C_4$-, besonders bevorzugt $C_1$-$C_4$-Alkyl, Vinyl, 3-Aminopropyl, insbesondere Methyl oder Ethyl eingesetzt. Im speziellen bevorzugt ist R eine Methyl-Gruppe.

An der Oberfläche von reinen $[SiO_{4/2}]$-Gelen besitzt jedes Siliciumatom bei vollständiger Kondensation der kondensationsfähigen Si-OH-Gruppen mindestens eine freie Si-OH Gruppe. Diese freien OH-Gruppen an der Oberfläche der Gele stehen für die Oberflächenmodifizierung (Silylierung) zur Verfügung, d.h. zur Reaktion mit einem Silyliermittel.

Bevorzugt liegt der molare Anteil an oxidischen Einheiten im gesamten aus den Primärpartikeln aufgebauten Gelnetzwerk (d.h. vor einer potentiellen Oberflächenmodifizierung/Silylierung des Gels) bei mehr als 1 %, besonders bevorzugt bei über 50%, insbesondere bevorzugt bei über 80%, und im speziellen bevorzugt bei über 90%. Das molare Verhältnis der oxidischen Einheiten zu $[R_xSiO_{(4-x)/2}]$-Einheiten ist durch die eingesetzten Mengen der betreffenden Ausgangsstoffe einfach steuerbar.

Als monofunktionelle Einheiten werden im Sinne der Erfindung aus der Nomenklatur der Silicone bekannte M-Einheiten verstanden, das heißt $[R_3SiO_{1/2}]$-Einheiten, wobei R gleich oder verschieden sein kann und für R die eingangs gegebene Definition gilt. Bevorzugt ist R gleich, besonders bevorzugt ist R eine Methylgruppe. Die auf Methyl basierende, monofunktionelle Einheiten $[(CH_3)_3SiO_{1/2}]$-Einheit ist somit eine Trimethylsiloxy-Einheit. Eingeführt werden können monofunktionelle Einheiten durch dem Fachmann bekannte Silyliermittel, wie beispielsweise Trimethylchlorsilan, Hexamethyldisilazan oder Hexamethyldisiloxan.

Der Belegungsgrad mit TMS-Gruppen für durchgehend hydrophobe $[SiO_{4/2}]$-Aerogele ist in EP 0 948 395 B1 mit mindestens 2,6 nm$^{-2}$ angegeben, was mit einem Kohlenstoffgehalt von minimal 6,8 Gew.-% korrespondiert.

Gele, die durchgehend aus $[R_xSiO_{(4-x)/2}]$-Einheiten (mit x=1 und/oder 2) aufgebaut sind, besitzen bei vollständiger Kondensation der kondensationsfähigen Si-OH-Gruppen dagegen keine freien Si-OH-Gruppen an der Oberfläche. Der C-Gehalt des Gels wird also ausschließlich durch den Rest R in den $[R_xSiO_{(4-x)/2}]$-Einheiten festgelegt, eine nachträgliche Silylierung kann hier bei vollständiger Kondensation nicht mehr erfolgen.

Daher ist eine möglichst vollständige Kondensation der kondensationsfähigen freien, für eine Silylierung zugänglichen OH-Gruppen für einen geringen Belegungsgrad an monofunktionellen Einheiten von Vorteil. Das bedeutet, dass das Vorliegen möglichst weniger, für die nachfolgende Silylierung zur Verfügung stehender freier für eine Silylierung zugänglichen OH-Gruppen vorteilhaft ist. Durch den erfindungsgemäßen Aufbau der Primärpartikel wird genau das erreicht: die Anzahl der zur Silylierung und somit durchgehenden Hydrophobierung zugänglichen OH-Gruppen wird durch den Einbau von Si-R-Gruppen (bzw. $[R_xSiO_{(4-x)/2}]$-Einheiten mit x=1 und/oder 2 und R ist gleich oder verschieden und R ist Wasserstoff oder ein organischer, substituierter oder unsubstituierter Rest) stark reduziert, so dass man nach Silylierung einen im Vergleich zu einem konventionellen Aufbau der Primärpartikel reduzierten Belegungsgrad findet.

Zum Erreichen eines möglichst geringen C-Gehalts ist es von Vorteil, wenn die Reste R der $[R_xSiO_{(4-x)/2}]$-Einheiten (mit x=1 und/oder 2 und R ist gleich oder verschieden und R ist Wasserstoff oder ein organischer, substituierter oder unsubstituierter Rest) möglichst wenige C-Atome aufweisen. Ebenso ist es von Vorteil, wenn das Silyliermittel Gruppen mit möglichst wenigen C-Atomen einführt. Bevorzugt ist die Anzahl der C-Atome in den erfindungsgemäßen

[$R_x$SiO$_{(4-x)/2}$]-Einheiten geringer, als die Anzahl der C-Atome im Silyliermittel. Besonders bevorzugt handelt es sich bei den mittels Modifizierungsreaktion eingeführten Gruppen um TMS-Gruppen.

Wenn es sich bei der eingeführten monofunktionellen Gruppe um TMS handelt, das bei der Oberflächenmodifizierung drei C-Atome je zugänglicher, nicht kondensierter OH-Gruppe einführt, ist es besonders vorteilhaft, wenn die [$R_x$SiO$_{(4-x)/2}$]-Einheiten entsprechend der vorliegenden Erfindung 1-2 C-Atome besitzen. Da (CH$_3$)$_3$SiO$_{1/2}$-Gruppen (TMS-Gruppen) 44,4 Gew.-% Kohlenstoff, [(CH$_3$)$_2$SiO$_{2/2}$]-Einheiten jedoch nur 32,4 Gew.-% Kohlenstoff und [(CH$_3$)SiO$_{3/2}$]-Einheiten lediglich 17,8 Gew.-% Kohlenstoff enthalten, zeichnen sich die erfindungsgemäß aufgebauten Gele, im Vergleich zu konventionellen, mit TMS-Gruppen gesättigten [SiO$_{4/2}$]-Gelen, durch einen verminderten Kohlenstoffgehalt aus. Der C-Gehalt ist besonders gering, wenn der oxidische Kern der Gele möglichst vollständig mit [$R_x$SiO$_{(4-x)/2}$]-Einheiten umhüllt ist, wobei der molare Anteil an [$R_x$SiO$_{(4-x)/2}$]-Einheiten im gesamten Gelnetzwerk vor der Silylierung bevorzugt höchstens 99 %, besonders bevorzugt höchstens 50 %, darüber hinaus bevorzugt höchstens 20 % und insbesondere bevorzugt höchstens 10 % beträgt. Wenn es sich um Aerogele handelt, liegt der Kohlenstoffgehalt der erfindungsgemäßen Gele bevorzugt unter 15 Gew.-%, besonders bevorzugt unter 10 Gew.-% und insbesondere bevorzugt unter 5 Gew.-%.

Die erfindungsgemäßen Netzwerke aus optimierten Primärpartikeln weisen bevorzugt einen Belegungsgrad mit monofunktionellen Einheiten, insbesondere bevorzugt mit TMS-Gruppen, von unter 1,5 Gruppen pro nm$^2$, besonders bevorzugt unter 1,0 Gruppe pro nm$^2$, insbesondere bevorzugt unter 0,5 Gruppen pro nm$^2$ und im speziellen bevorzugt von 0,2 Gruppen pro nm$^2$ auf. In einer darüber hinaus bevorzugten Ausführungsform tragen die Gele keine monofunktionellen Einheiten.

Wie Vergleichsbeispiel 1 zeigt, wurde durch klassische Synthese eines hydrophoben SiO$_2$-Aerogels aus Wasserglas nach Silylierung ein Gel mit einem TMS-Gruppen Belegungsgrad von 2,70 nm$^{-2}$ hergestellt. Dagegen wies ein reines [CH$_3$SiO$_{3/2}$]-Aerogel (s. Vergleichsbeispiel 3) nach Silylierung einen TMS-Gruppen-Belegungsgrad von 0,22 nm$^{-2}$ auf. Der Vergleich dieser beiden Experimente zeigt deutlich, dass bei einem geringeren Anteil an freien Si-OH-Gruppen, wie es in den [RSiO$_{3/2}$]-Gelen der Fall ist, nach der Silylierung auch eine deutlich geringere Belegung mit TMS-Gruppen resultiert.

Der Aufbau von Netzwerken aus optimierten Primärpartikeln verhindert den direkten, herstellungsbedingt starren Kontakt von [SiO$_{4/2}$]-Partikeln über [SiO$_{4/2}$]-Brücken. Dem Fachmann ist aus der Literatur bekannt, dass Netzwerke mit Kontakten aus [$R_x$SiO$_{(4-x)/2}$]-Einheiten mit x = 1 und/oder 2 flexible Strukturen liefern (Rao et al. 2006, s.o. und K. Kanamori et al. 2007 s.o.).

Die erfindungsgemäßen Gelnetzwerke enthalten die beschriebenen, optimierten Primärpartikeln. Bevorzugt enthalten die erfindungsgemäßen Gele nur die optimierten Primärpartikel. Die Verbindungen zwischen den Primärpartikeln sind durch Sinterhälse stabilisiert, wobei diese einen mit der äußeren Hülle der Primärpartikel vergleichbar hohen Anteil an [$R_x$SiO$_{(4-x)/2}$]-Einheiten aufweisen und bevorzugt nur aus [$R_x$SiO$_{(4-x)/2}$]-Einheiten bestehen. Durch den erfindungsgemäßen Aufbau und Verknüpfung der Primärpartikel mit [$R_x$SiO$_{(4-x)/2}$]-Einheiten sind somit flexible Aerogele zugänglich. Die Kombination aus Flexibilität und geringem Kohlenstoffgehalt ist ein wesentlicher, struktureller Vorteil der erfindungsgemäßen Strukturen.

Aufgebaut in dieser Form sind die erfindungsgemäßen Lyogele, bei denen die Poren mit einer Flüssigkeit gefüllt sind und die erfindungsgemäßen Aerogele, welche nach Trocknung aus Lyogelen erhalten werden und deren Poren mit Luft gefüllt sind.

Wenn es sich bei den Gelen um Aerogele handelt, liegt die Dichte des Gels bevorzugt unter 0,25 g/cm$^3$, besonders bevorzugt unter 0,15 g/cm$^3$ und insbesondere bevorzugt unter 0,1 g/cm$^3$.

Daher handelt es sich um einen bevorzugten Gegenstand der Erfindung, wenn es sich bei den Gelen um Aerogele handelt und die Dichte unter 0,15 g/cm$^3$ liegt, der Belegungsgrad an monofunktionellen Einheiten höchstens 1 pro nm$^2$ beträgt, der Kohlenstoffgehalt höchstes 8 Gew.-% beträgt und die BET-Oberfläche größer 300 m$^2$/g ist.

Vergleichsbeispiel 2 stellt die Cokondensation von Wasserglas und Kaliummethylsiliconat dar, es wurde nach Silylierung ein TMS-Gruppen Belegungsgrad von 1,57 nm$^{-2}$ erreicht.

Das erfindungsgemäße Beispiel 1 stellt die sequentielle Kondensation von Wasserglas und Kaliummethylsiliconat zum Aufbau eines Gels nach einem Gradientenmodell dar. Es wurde nach Silylierung ein TMS-Gruppen Belegungsgrad von 0,71 nm$^{-2}$ erreicht. Der deutlich geringere Belegungsgrad mit TMS-Gruppen im Vergleich zu reinen SiO$_2$-Gelen (Vergleichsbeispiel 1) und den nicht erfindungsgemäßen Gelen aus der Cokondensation (Vergleichsbeispiel 2) belegt die Anreicherung der [RSiO$_{3/2}$]-Einheiten mit von innen nach außen zunehmender Konzentration. Dieser erfindungsgemäße Aufbau führte zu einer signifikanten Verringerung der TMS-Gruppen-Belegung.

Beispiel 2 stellt ein Gel zur Verfügung, das in Form des Kern-Schale-Modells aufgebaut ist. Als [SiO$_{4/2}$]-Einheiten wurden kolloidale SiO$_2$-Partikel eingesetzt und diese mit [RSiO$_{3/2}$]-Einheiten aus Kaliummethylsiliconat belegt und zu einem Gelnetzwerk verbunden. Es wird nach Silylierung ein TMS-Gruppen Belegungsgrad von 0,11 nm$^{-2}$ erreicht. Beispiel 2 zeigt, dass durch den Aufbau von Primärpartikeln im Sinne von Kern-Schale-Partikeln und deren Versinterung zu Netzwerken ein Belegungsgrad an TMS-Gruppen in der Größenordnung reiner [RSiO$_{3/2}$]-Gele (vgl. Vergleichsbeispiel 3) erreicht werden kann. Dies ist zugleich der Nachweis dafür, dass die aus reinem SiO$_2$ bestehenden Kerne der Partikel vollständig mit [RSiO$_{3/2}$]-Einheiten belegt und die Primärpartikel auch über [RSiO$_{3/2}$]-Sinterbrücken verbunden sind. Bei Vorliegen von nicht kondensierten Si-OH Gruppen, wie sie bei einer Oberfläche aus reinen SiO$_2$-Einheiten auftreten, würde man einen erhöhten Belegungsgrad mit TMS-Gruppen wiederfinden.

Ein weiterer Gegenstand der Erfindung ist ein Verfahren zur Herstellung der erfindungsgemäßen Gele, indem i) ein Sol enthaltend kolloidale Partikel, wobei die kolloidalen Partikel oxidische Einheiten enthalten, vorgelegt wird, wobei die oxidischen Einheiten [SiO$_{4/2}$]-Einheiten enthalten und wobei die Sole durch Neutralisation von stark basischen Alkalisilikaten oder durch Hydrolyse von Alkoxysilanen hergestellt werden, und ii) die Partikel im Sol mit [R$_x$SiO$_{(4-x)/2}$]-Einheiten zur Reaktion gebracht werden, wobei x gleich oder verschieden sein kann und 1 oder 2 ist und R gleich oder verschieden sein kann und Wasserstoff oder ein organischer, substituierter oder unsubstituierter Rest ist, und iii) aus den Partikeln ein Gel bildet.

Erfindungsgemäß wird zunächst ein Sol enthaltend kolloidale Partikel, wobei die kolloidalen Partikel oxidische Einheiten enthalten und wobei die oxidischen Einheiten [SiO$_{4/2}$]-Einheiten enthalten, vorgelegt. Das bedeutet, dass im 1. Schritt des Verfahrens (i) ein Sol enthaltend [SiO$_{4/2}$]-Einheiten hergestellt wird.

Unter einer Solherstellung versteht man das Mischen von kolloidalen Partikeln oder deren Ausgangsstoffe mit mindestens einem Lösungsmittel bzw. Dispergiermittel und gegebenenfalls weiteren Zusatzstoffen. Dabei kann während und/oder nach dem Mischen auch eine Reaktion der Ausgangsstoffe stattfinden. Sole aus Alkoxysilanen werden durch Hydrolyse unter Freisetzung der entsprechenden Alkohole hergestellt. Die Hydrolyse kann durch Zugabe von Säure und/oder Temperaturerhöhung beschleunigt werden. Sole aus Wassergläsern und/oder Siliconaten werden durch Neutralisation der stark basischen Alkalisilikate bzw. Alkysiliconate hergestellt. Dies kann nach dem Fachmann bekannten Methoden wie z.B. in EP 0 948 395 B beschrieben, durch Neutralisation mit Mineralsäuren und mittels saurer Ionenaustauscherharzen erfolgen.

Als oxidische Einheiten können [SiO$_{4/2}$]-Einheiten mit allen dem Fachmann bekannten, hydrolysestabilen Metalloxiden oder deren Mischungen eingesetzt werden, bevorzugt werden zusätzlich zu den [SiO$_{4/2}$]-Einheiten als oxidische Einheiten drei- oder vierwertige Einheiten, besonders bevorzugt werden nur silicatische Einheiten eingesetzt.

Als Ausgangsstoff für die Bildung von oxidischen Einheiten können alle dem Fachmann bekannten, kondensationsfähigen Metallalkoxide, Alkalisalze, Halogenidsalze oder weitere organische oder anorganische Ausgangsstoffe eingesetzt werden. Ebenso können kolloidale Partikel basierend auf den jeweiligen Metalloxiden oder deren Mischungen eingesetzt werden.

Als Ausgangsstoff für die Bildung von [SiO$_{4/2}$]-Einheiten ([SiO$_{4/2}$]-Ausgangsstoff) können dem Fachmann bekannte, kondensationsfähige tetra- oder höherfunktionelle Silane, Alkoxy silane, Alkylsilicate, Alkalisilicate oder kolloidale Silica-Partikel bzw. -Lösungen verwendet werden.

Es ist bevorzugt, als Ausgangsstoff für [SiO$_{4/2}$]-Einheiten Verbindungen das Typs Si(OR)$_4$, [SiO$_{4/2}$]$_w$[SiO$_{3/2}$(OR)]$_x$[SiO$_{2/2}$(OR)$_2$]$_y$[SiO$_{1/2}$(OR)$_3$]$_z$ (mit w, x, y, z als nicht negative, ganze Zahl), SiCl$_4$, Wassergläser oder kolloidale Silica-Lösungen einzusetzen. Für R gilt die bereits gegebene Definition.

Insbesondere bevorzugt wird Natriumwasserglas und/oder kolloidale Silica-Lösung eingesetzt. Im speziellen bevorzugt werden kolloidale Silica-Lösungen eingesetzt, wobei der mittlere Partikeldurchmesser der Silica-Partikel bevorzugt unter 8 nm, besonders bevorzugt unter 6 nm, insbesondere bevorzugt im Bereich von 1 bis 5 nm liegt. Die Partikel haben bevorzugt einen mittleren Durchmesser im Bereich von 1 bis 8 nm. Es können auch Gemische oder Hydrolyseprodukte der genannten Ausgangsstoffe, insbesondere deren Hydrolyseprodukte mit Wasser und/oder Alkoholen, eingesetzt werden.

Als Wasserglas werden aus einer Schmelze erstarrte, glasartige, also amorphe, wasserlösliche Natrium-, Kalium- und Lithiumsilicate oder ihre wässrigen Lösungen bezeichnet. Durch Neutralisieren des Salzes und Hydrolyse entstehen aus den kettenartigen Si-O-Si-Verbindungen [SiO$_{4/2}$]-Einheiten.

Erfindungsgemäß werden anschließend die Partikel im Sol mit [R$_x$SiO$_{(4-x)/2}$]-Einheiten zur Reaktion gebracht. Zur Reaktion bringen bedeutet im Rahmen dieser Erfindung, dass die Ausgangsstoffe zur Bildung von [R$_x$SiO$_{(4-x)/2}$]-Einheiten unter Mischen zum Sol gegeben werden, wobei eine chemische Umsetzung stattfindet.

Als Ausgangsstoff für die Bildung von [R$_x$SiO$_{(4-x)/2}$]-Einheiten ([R$_x$SiO$_{(4-x)/2}$]-Ausgangsstoff) können dem Fachmann bekannte, kondensationsfähige bifunktionelle, trifunktionelle oder höher funktionelle Silane, Alkoxysilane oder Siliconate eingesetzt werden. Gegebenenfalls können auch monofunktionelle Silane, Alkoxysilane oder Siliconate eingesetzt werden.

Bevorzugt eingesetzt werden Verbindungen des Typs RSi(OR)$_3$, RSiCl$_3$, [RSi(OH)$_{3-n}$(OM)$_n$] (mit n als nicht negative, ganze Zahl zwischen 0 und 3, mit M = Li, Na, K) sowie deren Hydrolyse und/oder Kondensationsprodukte, [[RSiO$_{3/2}$]$_x$[RSiO$_{2/2}$(OR)]$_y$[RSiO$_{1/2}$(OR)$_2$]$_z$ (mit x, y, z als nicht negative, ganze Zahl), R$_2$Si(OR)$_2$, R$_2$SiCl$_2$, [R$_2$Si(OH)$_{2-m}$(OM)$_m$] (mit m als nicht negative, ganze Zahl zwischen 0 und 2, mit M = Li, Na, K) sowie deren Hydrolyse und/oder Kondensationsprodukte, [R$_2$SiO$_{2/2}$]$_y$[R$_2$SiO$_{1/2}$(OR)]$_z$ (mit y, z als nicht negative, ganze Zahl), R$_3$SiCl, R$_3$SiOR, R$_3$Si-O-SiR$_3$, R$_3$Si-NH-SiR$_3$, R$_3$SiOH, R$_3$SiOM (mit M = Li, Na, K).

Besonders bevorzugt eingesetzt werden Methyltrialkoxysilane Vinyltrialkoxysilane, Dimethyldialkoxysilane, OH-, OR-, H- oder Cl-terminierte Polydimethylsiloxane, Alkalimethylsiliconate.

Es ist insbesondere bevorzugt, als Ausgangsstoff für die [R$_x$SiO$_{(4-x)/2}$]-Einheiten Methyltriethoxysilan (MTES), Methyltrimethoxysilan, Kaliummethylsiliconat oder Natriummethylsiliconat einzusetzen. Es können auch Gemische, Hydrolyseprodukte und/oder Kondensationsprodukte der genannten Ausgangsstoffe, insbesondere deren Hydrolyseprodukte mit Wasser und/oder Alkoholen, eingesetzt werden.

Bevorzugt ist mindestens ein Rest R der [R$_x$SiO$_{(4-x)/2}$]-Einheiten organischer Natur, d.h. es liegt mindestens eine Si-C-Bindung vor, besonders bevorzugt ist mindestens ein Rest R eine Methylgruppe, insbesondere bevorzugt sind alle Reste R der [R$_x$SiO$_{(4-x)/2}$]-Einheiten Methylgruppen.

Die erfindungsgemäßen Gele enthalten die zuvor beschriebenen optimierten Primärpartikeln. Daher kann die Herstellung der erfindungsgemäßen Gele auch durch gleichzeitige Zugabe optimierter Primärpartikel und weiterer Partikel, beispielsweise basierend auf [SiO$_{4/2}$]-Einheiten oder [R$_x$SiO$_{(4-x)/2}$]-Einheiten oder deren homogenen Mischungen, durchgeführt werden. Bevorzugt bestehen die erfindungsgemäßen Gele nur aus den optimierten Primärpartikeln, daher ist es zweckmäßig, keine Mischungen an Partikeln zu Gelen umzusetzen, sondern durch das Verfahren nur die optimierten Primärpartikel zur Verfügung zu stellen.

Der Aufbau von optimierten Primärpartikeln kann durch sequentielle, also zeitlich versetzte, Kondensation von oxidischen Einheiten und [R$_x$SiO$_{(4-x)/2}$]-Einheiten durchgeführt werden.

Der Aufbau der bevorzugten, optimierten Primärpartikel nach dem Gradientenaufbau kann durch sequentielle, also zeitlich versetzte Kondensation von oxidischen Einheiten, aus denen ein partikelhaltiges Sol mit möglichst geringem Anteil an verbleibenden Monomeren und/oder Oligomeren gebildet wird, und [R$_x$SiO$_{(4-x)/2}$]-Einheiten, aus denen hauptsächlich die Schale der Primärpartikel und die Sinterbrücken gebildet werden, realisiert werden. Dabei ist es vorteilhaft, dass die vorgelegten Ausgangsstoffe für den an [R$_x$SiO$_{(4-x)/2}$]-Einheiten armen Kern vor der Zugabe der Ausgangsstoffe für die [R$_x$SiO$_{(4-x)/2}$]-reiche Schale bereits weitgehend zum Sol umgesetzt wurden. Durch die zeitlich versetzte Zugabe der [R$_x$SiO$_{(4-x)/2}$]-Ausgangsstoffe findet eine Anreicherung in Form des zuvor beschriebenen Gradienten nach außen statt (s. Fig. 3). Es ist jedoch auch möglich, die Ausgangsstoffe in Mischungen oder in anderer Reihenfolge zum Sol umzusetzen, so dass ein Minimum an [R$_x$SiO$_{(4-x)/2}$]-Einheiten durchlaufen werden kann oder auch im Primärpartikel bereits eine Mischung aus [R$_x$SiO$_{(4-x)/2}$]-Einheiten und oxidischen Einheiten vorliegt.

Der Aufbau der ebenfalls bevorzugten, optimierten Primärpartikel nach dem Kern-Schale-Modell kann durch das Vorlegen von isolierten, oxidischen Nanopartikeln unter Abwesenheit von monomeren oder oligomeren Einheiten in Lösung und anschließender Zugabe von [R$_x$SiO$_{(4-x)/2}$]-Ausgangsstoffen durchgeführt werden. In dieser Ausführungsform können beispielsweise kolloidale Suspensionen von Nanopartikeln basierend auf Mischungen verschiedener Metalloxide eingesetzt werden, bevorzugt werden [SiO$_{4/2}$]-Partikel eingesetzt. Nach dieser Methode ist eine Anreicherung der [R$_x$SiO$_{(4-x)/2}$]-Einheiten nach außen in Form eines Kern-Schale-Aufbaus möglich, bei dem im Inneren des Primärpartikels nur oxidische, bevorzugt [SiO$_{4/2}$]-Einheiten, und außen und an den Sinterhälsen nur [R$_x$SiO$_{(4-x)/2}$]-Einheiten vorliegen (siehe Fig. 4).

Kolloidale Partikel bezeichnen Teilchen oder Tröpfchen, die im Dispersionsmedium (Feststoff, Gas oder Flüssigkeit) fein verteilt sind. Die Größe der einzelnen Teilchen liegt typischerweise im Nanometer- oder Mikrometer-Bereich. Erfindungsgemäß enthalten die kolloidalen Partikel [SiO$_{4/2}$]-Einheiten, entweder in Mischung mit anderen Metalloxiden oder als reines SiO$_2$. Bevorzugt liegt der mittlere Durchmesser der Partikel im Bereich von 1 bis 8 nm, besonders bevorzugt im Bereich von 1 bis 6 nm und insbesondere bevorzugt im Bereich von 1 bis 5 nm.

Für die Herstellung von erfindungsgemäßen Primärpartikeln mit einem gradientenartigen Aufbau oder einem Kern-Schale Aufbau sowie Solen enthaltend kolloidal gelöste Primärpartikel mit dem beschriebenen Aufbau erfolgt eine zeitlich versetzte, also spätere Zugabe von mindestens einem Teil der [R$_x$SiO$_{(4-x)/2}$]-Ausgangsstoffe. Durch diese zeitlich versetzte Zugabe von mindestens einem Teil der [R$_x$SiO$_{(4-x)/2}$]-Ausgangsstoffe in das noch flüssige Sol gelingt eine Anreicherung der [R$_x$SiO$_{(4-x)/2}$]-Einheiten an der Oberfläche der Sol-Partikel und damit ein Gradienten-artiger oder Kern-Schale-artiger Aufbau der Primärpartikel.

Dabei ist es vorteilhaft, die vorgelegten Ausgangsstoffe vor der Zugabe der Ausgangsstoffe für [R$_x$SiO$_{(4-x)/2}$] bereits weitgehend zum Sol umzusetzen. Eine gründliche Umsetzung kann durch eine Temperaturerhöhung und/oder Wartezeit erreicht werden. Eine spätere, zeitlich versetzte Zugabe von mindestens einem Teil der [R$_x$SiO$_{(4-x)/2}$]-Ausgangsstoffe in das noch flüssige Sol bedeutet im Rahmen dieser Erfindung, dass nach der Zugabe der ersten Ausgangsstoffe bevorzugt 5 Minuten bis 10 Stunden, besonders bevorzugt 30 Minuten bis 5 Stunden, insbesondere bevorzugt 30 Min bis 2 Stunden unter weiterem Rühren inkubiert wird, bevor die restlichen Ausgangsstoffe für [R$_x$SiO$_{(4-x)/2}$] zugegeben werden. Dabei wird das flüssige Sol bevorzugt auf eine Temperatur zwischen 5 und 100 °C, besonders bevorzugt zwischen 10 und 80 °C, insbesondere bevorzugt zwischen 15 und 40 °C temperiert. Es ist besonders bevorzugt als oxidischen Ausgangsstoff kolloidale Nanopartikel basierend auf Metalloxiden enthaltend [SiO$_{4/2}$]-Einheiten, einzusetzen. Bevorzugt werden reine [SiO$_{4/2}$]-Nanopartikel eingesetzt. Die Verwendung kolloidaler Nanopartikel ist besonders vorteilhaft, weil dadurch sichergestellt werden kann, dass die oxidischen Ausgangsstoffe bereits vollständig zu einem kolloidalen Sol umgesetzt sind.

Eine bevorzugte Ausführungsform des Verfahrens ist es, im Schritt der Solherstellung (Schritt i) mindestens 1 Gew.-%, des Weiteren bevorzugt mindestens 25 Gew.-%, besonders bevorzugt mindestens 50 Gew.-% und insbesondere bevorzugt mindestens 80 Gew.-% der [$R_x SiO_{(4-x)/2}$]-Ausgangsstoffe erst später zu den bereits vorgelegten Ausgangsstoffen zuzugeben. In einer besonders bevorzugten Ausführungsform wird der gesamte Anteil der [$R_x SiO_{(4-x)/2}$]-Ausgangsstoffe erst später zum Sol gegeben. Kolloidale Nanopartikel werden bevorzugt als kolloidale Lösung eingesetzt.

Der Anteil der [$R_x SiO_{(4-x)/2}$]-Einheiten (bezogen auf die Summe der oxidischen Einheiten und der [$R_x SiO_{(4-x)/2}$]-Einheiten) liegt im Bereich zwischen 1 und 99 mol-%, bevorzugt zwischen 1 und 50 mol-%, besonders bevorzugt zwischen 1 und 20 mol-% und insbesondere bevorzugt zwischen 1 und 10 mol-%. Im Allgemeinen beträgt der Feststoffgehalt, also der Gehalt an oxidischen Einheiten und [$R_x SiO_{(4-x)/2}$]-Einheiten, im Sol zwischen 3 und 30 Gew.-%, bevorzugt zwischen 5 und 20 Gew.-%, besonders bevorzugt zwischen 8 und 15 Gew.-%.

In einer besonders bevorzugten Ausführungsform werden [$SiO_{4/2}$]-Partikel und Säure vorgelegt und mit Kaliummethylsiliconat-Lösung zur Reaktion gebracht, wodurch die [$SiO_{4/2}$]-Partikel mit einer [$R_x SiO_{(4-x)/2}$]-Schale belegt werden. Die alkalische Kaliummethylsiliconat-Lösung dient hierbei sowohl als Ausgangsstoff für die [$R_x SiO_{(4-x)/2}$]-Einheiten als auch als Base, um die Gelbildung zu starten. Im Allgemeinen können aber alle der bereits beschriebenen Ausgangsstoffe für [$R_x SiO_{(4-x)/2}$]-Einheiten eingesetzt werden. Während der Sol-Herstellung kann das Reaktionsgemisch gegebenenfalls gekühlt werden. Die Gelbildung erfolgt nach dem Fachmann bekannten Methoden wie pH-Wert-Erhöhung und/oder Temperaturerhöhung.

Darüber hinaus können dem Sol Zusatzstoffe wie dem Fachmann bekannte IR-Trübungsmittel zur Reduzierung der Wärmeleitfähigkeit zugesetzt werden. Ebenso können zur Erhöhung der mechanischen Stabilität beschichtete und/oder unbeschichtete Fasern zugesetzt werden. Als Fasermaterialien können anorganische Fasern, wie z. B. Glasfasern oder Mineralfasern, organische Fasern, wie z. B. Polyesterfasern, Aramidfasern, Nylonfasern oder Fasern pflanzlichen Ursprungs, sowie Gemische derselben verwendet werden.

Ein weiteres Merkmal des Verfahrens ist, dass aus dem Sol ein Gel gebildet wird. Die Gelbildung kann während oder nach der Zugabe der Ausgangsstoffe zur Bildung von [$R_x SiO_{(4-x)/2}$]-Einheiten zum Sol erfolgen. D.h. die Punkte ii und iii sind nicht notwendigerweise aufeinander folgende Schritte, iii kann auch während ii erfolgen.

Für die Gelbildung wird bevorzugt ein pH-Wert von 4 bis 10, besonders bevorzugt zwischen 5 und 9, insbesondere bevorzugt zwischen 6 und 8 eingestellt. Hierzu können im allgemeinen alle dem Fachmann bekannten Basen wie $NH_4OH$, NaOH, KOH, $Al(OH)_3$ Silicate oder Siliconate eingesetzt werden, bevorzugt wird $NH_4OH$ (Ammoniak), Wasserglas oder Alkalimethylsiliconat eingesetzt. In einer besonders bevorzugten Ausführungsform wird Kaliummethylsiliconat als Base eingesetzt. Eine Beschleunigung der Gelbildungszeit kann auch über eine Temperaturerhöhung erreicht werden. Im Allgemeinen wird die Gelbildung bei einer Temperatur zwischen 0 °C und dem Siedepunkt der enthaltenden Lösemittel, bevorzugt zwischen 15 und 80 °C durchgeführt.

In einer bevorzugten Ausführungsform wird das Sol in Schritt ii), d.h. während der Belegung mit [$R_x SiO_{(4-x)/2}$]-Einheiten, in ein Gel überführt.

In einer alternativ bevorzugten Ausführungsform wird das Sol in Schritt im Anschluss an Schritt ii), d.h. nach der Belegung mit [$R_x SiO_{(4-x)/2}$]-Einheiten in ein Gel überführt.

Im Anschluss an die Gelbildung kann eine Alterung erfolgen, die ebenfalls nach bekannten Methoden wie pH-Kontrolle und Erhitzen beschleunigt werden kann. Eine Alterung im Sinne der Erfindung bedeutet, dass das Gel bei einer Temperatur im Bereich von 5 bis 100 °C, vorzugsweise bei 50 bis 80 °C und insbesondere bevorzugt bei 60 °C und einem pH-Wert von 4-11, vorzugsweise 7-10 und insbesondere 8-9 für eine definierte Zeitperiode (Alterungsdauer) inkubiert wird. Die Alterungsdauer ist für eine möglichst vollständige Kondensation der kondensationsfähigen, für eine Silylierung zugänglichen OH-Gruppen an der Oberfläche entscheidend. Diese kann gegebenenfalls mehrere Tage dauern. Bevorzugt liegt die Alterungsdauer zwischen 30 Minuten und 1 Woche, besonders bevorzugt zwischen 1 Stunde und 3 Tagen, insbesondere bevorzugt zwischen 3 und 48 Stunden. Das gebildete Lyogel kann vor, während oder nach einer gegebenenfalls erfolgten Alterung mit Wasser, polaren oder unpolaren organischen Lösemittels oder Gemischen davon gewaschen werden, um z.B. Elektrolyte zu entfernen oder die Porenflüssigkeit auszutauschen. Das Lyogel kann über Lösemittelaustauschschritte auch mit einem für die weitere Verwendung des Lyogels vorteilhaften Lösungsmitteln oder Lösungen von Wirkstoffen beladen werden. Dies ist beispielsweise bei der Verwendung der Lyogele für kosmetische Anwendungen relevant.

Gegebenenfalls wird das Gel anschließend oberflächenmodifiziert. Eine bevorzugte Oberflächenmodifizierung ist die Silylierung. Als Silylierungsmittel können alle dem Fachmann bekannten Silylierungsmittel wie Silane, Chlorsilane, Alkoxysilane, Siloxane, Silanole, Silazane sowie Gemische oder Hydrolyse- oder Spaltprodukte der genannten Silylierungsmittel eingesetzt werden. Besonders bevorzugt werden Silylierungsmittel eingesetzt, die zur Bildung monofunktioneller Einheiten führen. Das bedeutet, dass das Gel in dieser bevorzugten Ausführungsform durch Oberflächenmodifizierung mit monofunktionellen Einheiten belegt wird. Für monofunktionelle Einheiten gilt die gegebene Definition. Bevorzugt eingesetzt werden Verbindungen des Typs $R_3SiCl$, $R_3SiOH$, $R_3SiOR$, $R_3SiH$, $R_3Si-O-SiR_3$, $R_3Si-NH-SiR_3$, wobei für R die gegebene Definition gilt. Besonders bevorzugt eingesetzt werden Trimethylchlorsilan und/oder Hexamethyldisiloxan. Gegebenenfalls werden zum Start der Silylierungsreaktion auch Initiatoren wie Mineralsäuren, bevorzugt Salzsäure zugegeben. Die Zeit für den Prozess der Oberflächenmodifizierung beträgt bevorzugt weniger als 12 Stunden, weiter bevorzugt zwischen 15 Minuten und 3 Stunden, insbesondere bevorzugt zwischen 15 Minuten und 2 Stunden und speziell bevorzugt zwischen 15 und 60 Minuten. Die Reaktion wird bevorzugt zwischen 50 und 90 °C, besonders bevorzugt zwischen 60 und 80 °C durchgeführt. Gegebenenfalls können während der Oberflächenmodifizierung auch Hilfsstoffe wie Phasenvermittler anwesend sein.

Unter einem Phasenvermittler wird in der vorliegenden Anmeldung eine polare Verbindung oder Gemische verschiedener dieser Verbindungen verstanden, die sowohl in der wasserreichen Phase als auch in der organischen Phase eine merkliche Löslichkeit besitzt und somit den Stofftransport zwischen den beiden im Wesentlichen nicht mischbaren Phasen beschleunigt.

Als Phasenvermittler geeignet sind polare organische Verbindungen oder Gemische davon wie

- Alkohole, insbesondere der chemischen Formel R-OH, wobei R wie oben für Reste R angegeben definiert ist (z.B. Methanol, Ethanol, Isopropanol)
- Ketone, insbesondere der chemischen Formel $R^1R^2C=O$, wobei $R^1$ und $R^2$ gleich oder verschieden und wie oben für Reste R angegeben definiert sind (z.B. Aceton $(CH_3)_2C=O$)
- Ether, insbesondere der chemischen Formel $R^1OR^2$, wobei $R^1$ und $R^2$ gleich oder verschieden und wie oben für Reste R angegeben definiert sind (z.B. Diethylether, Tetrahydrofuran, Dimethoxyethan)
- Ester, insbesondere der chemischen Formel $R^1COOR^2$, wobei $R^1$ und $R^2$ gleich oder verschieden und wie oben für Reste R angegeben definiert sind (z.B. Ethylacetat) und
- grenzflächenaktive Substanzen wie Tenside. Grenzflächenaktiv nennt man organische Verbindungen, die sich dank ihrer Struktur in der Grenzfläche zwischen zwei Phasen so anordnen, dass sie die Grenzflächenspannung (=Oberflächenspannung) erniedrigen und dadurch z.B. Benetzung ermöglichen. Durch Herabsetzen der Oberflächenspannung fördern sie die Durchmischung von zwei Phasen unter Umständen bis zur Bildung einer Emulsion. Je nach ihrer chemischen Zusammensetzung und Anwendung werden grenzflächenaktive Substanzen als Netzmittel, Detergenzien (Tenside, Seife) oder Emulgatoren bezeichnet.
  Die Stoffe enthalten im Allgemeinen je eine Wasser stark anziehende hydrophile ("wasserfreundliche") Gruppe und eine lipophile ("fettfreundliche"), Wassermoleküle nur schwach anziehende (hydrophobe) Kohlenwasserstoffgruppe.

Anschließend kann das Gel gegebenenfalls gewaschen werden. Es ist bevorzugt, dass das nach dem erfindungsgemäßen Verfahren hergestellte Gel getrocknet wird. Im Allgemeinen kann die Trocknung sowohl im überkritischen Bereich als auch im unterkritischen Bereich erfolgen. Bevorzugt findet eine Trocknung unterhalb des kritischen Punktes statt, vorzugsweise bei Temperaturen von -30 bis 200 °C, besonders bevorzugt 0 bis 150 °C, sowie bei Drücken vorzugsweise von 0,001 bis 20 bar, besonders bevorzugt 0,01 bis 5 bar, insbesondere 0,01 bis 2 bar. Die Trocknung kann dabei durch Strahlungs-, Konvektions- und/oder Kontakttrocknung erfolgen. Die Trocknung wird vorzugsweise so lange durchgeführt, bis das Gel einen Lösungsmittel-Restgehalt von weniger als 0,1 Gew.-% aufweist.

Die erfindungsgemäßen Gele werden bevorzugt in kosmetischen, medizinischen oder für chromatographische Anwendungen oder als Katalysator oder Katalysatorträger verwendet. Wenn es sich bei den Gelen um Aerogele handelt, werden diese bevorzugt für Anwendungen zur thermischen und/oder akustischen Isolierung verwendet.

Wenn es sich bei den erfindungsgemäßen Gelen um Aerogele handelt, finden die erfindungsgemäßen Gele bevorzugt Verwendung in der dem Fachmann bekannten Anwendungsformen zur thermischen Isolierung, etwa als Komponente in anorganischen Putzsystemen, ggf. in Kombination mit geeigneten organischen und/oder anorganischen Bindersystemen, nach weiterer Verarbeitung in Form von Platten, welche direkt als Dämmstoff eingesetzt werden können oder als Füllstoff für Hohlbausteine verwendet werden können, oder als einsatzfertige Matten.

Wenn es sich bei den erfindungsgemäßen Gelen um Aerogele handelt, können diese des Weiteren als Materialien für akustische Isolierung, stationäre Phasen in der Flüssig- und Gaschromatographie, als Zusatzstoffe für kosmetische Anwendungen, als Katalysatoren oder Katalysatorträger, oder als hocheffektive Adsorptions- bzw. Absorptionsmittel, oder etwa im medizinischen Bereich als Wirkstoffträger eingesetzt werden. Die erfindungsgemäßen Aerogele, vorzugsweise solche mit funktionalisierten Oberflächen (z.B. Vinyl-, Epoxy-, Aminopropyl-) können auch als Additiv z.B. zur Einstellung der mechanischen Eigenschaften in Kunststoffen, insbesondere Kautschuken, Elastomeren eingesetzt werden.

Wenn es sich bei den erfindungsgemäßen Gelen um Lyogele handelt, können diese unter anderem Anwendung in Dispersionen, Emulsionen, Pasten und anderen Formulierungen für kosmetische Anwendungen, Schleif-, Putz- und Poliermitteln finden.

Die Abbildungen beschreiben die Erfindung beispielhaft, ohne sie zu beschränken.

**Fig. 1:** Schematische Darstellung der nachträglichen Modifizierung von bereits bestehenden $[SiO_{4/2}]$-Gelnetzwerken mit interpartikulären Verknüpfungen (Sinterbrücken) aus $[SiO_{4/2}]$-(A=Kern, helles Zentrum) mit $[R_x-SiO_{(4-x)/2}]$-Einheiten (B=Schale, schwarze Hülle). Da die interpartikulären Verknüpfungen (C) bereits vor der Modifizierung ausgebildet wurden, sind diese hell dargestellt.

**Fig. 2:** Schematische Darstellung eines aus [$R_xSiO_{(4-x)/2}$]-Einheiten bestehenden Gelnetzwerks. Das gesamte Gelnetzwerk, d.h. sowohl der Kern (A), die Schale oder Hülle (B) und die interpartikulären Verknüpfungen (C), bestehen aus [$R_xSiO_{(4-x)/2}$]-Einheiten,

**Fig. 3:** Schematische Darstellung des Gradientenaufbaus der Primärpartikel mit nach außen zunehmender [$R_xSiO_{(4-x)/2}$]-Konzentration und [$R_xSiO_{(4-x)/2}$]-reichen interpartikulären Verknüpfungen (Sinterbrücken, C), wobei das helle Zentrum oxidische Einheiten mit höchster Konzentration im Kern (A) und die von innen nach außen zunehmend dunkle Färbung die innen geringere und nach außen zunehmende Konzentration von [$R_xSiO_{(4-x)/2}$]-Einheiten wiederspiegelt. Die Sinterbrücken (C) weisen dieselbe Zusammensetzung wie die äußere Hülle der Primärpartikel (B) auf.

**Fig. 4:** Schematische Darstellung des Kern-Schale-Aufbaus der einzelnen Primärpartikel mit oxidischem Kern (A, helles Zentrum) und [$R_xSiO_{(4-x)/2}$]-Schale (B, schwarze Hülle) und interpartikuläre Verknüpfungen (C, Sinterbrücken) der einzelnen Primärpartikel. Da die Sinterbrücken dieselbe Zusammensetzung wie die Schalen aufweisen, sind diese schwarz dargestellt.

**Beispiele**

Die Beispiele erläutern die Erfindung näher, ohne ihren Umfang zu beschränken.

**Analysemethoden:**

Bestimmung der Dichte

Die Dichte der Aerogelstücke wurde mittels Pyknometrie bestimmt. Hierzu wurden die Aerogelstücke auf einer Analysenwaage gewogen ($m_1$) und zur Volumenbestimmung die Wasserverdrängung in einem 25 ml Pyknometer (Glaspyknometer nach Gay-Lussac nach DIN ISO 3507 der Firma Blaubrand) bei Raumtemperatur gemessen. Hierfür wurden folgende Massen auf einer Analysenwaage bestimmt:

$m_2$: Masse des Pyknometers gefüllt mit destilliertem Wasser
$m_3$: Masse des Pyknometers gefüllt mit dem Aerogelstück und destilliertem Wasser

Das Volumen des Aerogelstücks ($V_1$) entspricht dem Volumen des verdrängten Wassers ($V_2$). Das Volumen und die Dichte des Aerogelstücks wurden nach folgenden Formeln berechnet:

$$V_1 = V_2 = \rho_w * (m_2 - (m_3 - m_1))$$

$$\rho_{Aerogel} = m_1 / V_1$$

wobei $\rho_w$ die Dichte von Wasser bei Raumtemperatur (0,998 g/cm$^3$) angibt.
Bei dem Befüllen des Pyknometers mit dem Aerogelstück und dem Wasser wurde darauf geachtet, dass keine Luftblasen eingeschlossen wurden. Aufgrund der hohen Hydrophobie der Aerogelproben ist ein Eindringen von Wasser in die Poren der Proben ausgeschlossen. Zur Kontrolle wurde das Gewicht der Aerogelstücke nach der Messung durch erneutes Wiegen bestätigt.

Bestimmung der BET-Oberfläche

Die spezifische Oberfläche der Aerogele wurde bestimmt nach der BET-Methode entsprechend DIN 9277/66131 und 9277/66132).

Bestimmung des Kohlenstoffgehalts

Die Bestimmung des Kohlenstoffgehalts (C-Gehalts) der Proben wurde an einem Leco CS 230 Analysator durchgeführt. Die Analyse erfolgte durch Hochfrequenzverbrennung der Probe im Sauerstoffstrom. Detektiert wurde mittels nicht dispersiver Infrarotdetektoren.

Bestimmung des pH-Werts

Der pH-Wert wurde mit einem pH-Meter der Firma Mettler Toledo Seven Multi; Elektrode: In Lab Science ermittelt.

Berechnung des TMS-Gruppen-Belegungsgrads

Die TMS-Gruppenbelegung nach der Oberflächenmodifizierung wurde analog EP 0 948 395 B1 mittels folgender Formel berechnet:

Belegungsgrad = ($[C_{mit\ TMS}]$ - $[C_{ohne\ TMS}]$) /BET) * K; Einheit: [nm$^{-2}$] K = 6, 022*1023/100*12*3*1018 = 167,28; Einheit: [g$^{-1}$] $[C_{mit\ TMS}]$ : C-Gehalt nach der Oberflächenmodifizierung in Gew.-% $[C_{ohne\ TMS}]$ : C-Gehalt vor der Oberflächenmodifizierung in Gew.-% [BET]: BET-Oberfläche; Einheit: [m$^2$/g]

$$\Delta C\ [Gew.\text{-}\%]\ =\ C_{mit\ TMS}\ [Gew.\text{-}\%]\ -\ C_{ohne\ TMS}\ [Gew.\text{-}\%]$$

**Beispiele:**

**Bezugsquellen:**

Wasserglas (Sigma-Aldrich: $SiO_2$-Gehalt: 26,5 Gew.-%, $Na_2O$-Gehalt: 10,6 Gew.-%)
Kaliummethylsiliconat (SILRES® BS 16 der Wacker Chemie AG: wässrige Lösung mit 34 Gew.-% Wirkstoffanteil und 20 Gew.-% $K_2O$)
$SiO_2$-Nanosol (Bindzil 17/750 der Firma Akzo Nobel: $SiO_2$-Gehalt: 15 Gew.-%, mittl. Partikeldurchmesser laut Hersteller: 4 nm, pH 10,5)
Hexamethyldisiloxan (AK 0,65 der Wacker Chemie AG) Trimethylchlorsilan (SILAN M3 der Wacker Chemie AG) Methyltrimethoxysilan (Sigma-Aldrich, Grade: 98%) Cetyltrimethylammoniumbromid (Sigma-Aldrich) Alle weiteren Laborchemikalien, soweit nicht gesondert erwähnt, wurden von Sigma-Aldrich bezogen.

**Beispiel 1: Herstellung eines Aerogels aus Wasserglas und Kaliummethylsiliconat (durch sequentielle Zugabe von Kaliummethylsiliconat)**

In einem Becherglas wurden 55,5 g Wasser und 55,5 g Wasserglas gemischt und in einem Eisbad auf 10 °C gekühlt. In einem zweiten Becherglas wurden 55,5 g Wasser und 55,5 g Kaliummethylsiliconat gemischt und in einem Eisbad auf 10 °C gekühlt.
In einer Schraubflasche wurden 200 g Salzsäure (7,5 Gew.-%) vorgelegt, in einem Eisbad auf unter 10 °C gekühlt und mit einem Magnetrührer bei 500 UpM gerührt.
Die gekühlte Wasserglas-Lösung wurde über einen Tropftrichter langsam unter Rühren zu der Salzsäurelösung gegeben. Bei der Dosierung wurde darauf geachtet, dass die Tropfgeschwindigkeit so langsam eingestellt wurde, dass die Temperatur nicht über 10 °C steigt. Nach der Zugabe wurde das Reaktionsgemisch für zwei Stunden bei Raumtemperatur weiter gerührt und vor der Zugabe der zweiten Komponente wieder in einem Eisbad auf unter 10 °C gekühlt. Anschließend wurde die ebenfalls in einem Eisbad auf unter 10 °C gekühlte Kaliummethylsiliconat-Lösung über einen Tropftrichter langsam unter Rühren zugegeben, wobei bei der Dosierung darauf geachtet wurde, dass die Temperatur nicht über 10 °C steigt. Anschließend wurde das Rühren beendet und das Sol auf Raumtemperatur erwärmt, wodurch Gelbildung zum Lyogel stattfand.
Für die Alterung wurde das erhaltene Lyogel in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten.
Zur Entfernung der Salze wurden die Gelstücke fünfmal für jeweils 24 Stunden in 60 °C heißem, schwach alkalischem Wasser inkubiert (300 ml Wasser je 100 g Gel). Schwach alkalisch bedeutet, dass das Wasser mit NaOH auf einen pH-Wert von 8,5 eingestellt wurde. Das Wasser wurde dabei nach jeweils 24 Stunden durch Dekantieren abgetrennt und anschließend durch frisches, schwach alkalisches Wasser ersetzt.
Eine Probe von 10 g des erhaltenen feuchten Lyogels wurde zur Bestimmung des C-Gehalts vor der Oberflächenmodifizierung bei 180 °C in einem Trockenschrank bis zur Gewichtskonstanz getrocknet und anschließend wie oben beschrieben analysiert.
Parallel wurden 100 g des erhaltenen feuchten Gels vor der Oberflächenmodifizierung mit 200 ml einer Ethanol-Wasser-Mischung (50 Gew.-% Ethanol) überschichtet und für 16 Stunden bei Raumtemperatur in einem geschlossenen Gefäß inkubiert. Anschließend wurde das Gel mittels Filtration über einen Büchner Trichter (Whatman® Filter, 125 mm, Grade 40) abgetrennt. Für die Oberflächenmodifizierung wurden die erhaltenen Gelstücke in einer verschlossenen Schraubflasche mit 200 ml Hexamethyldisiloxan und 10,0 g Trimethylchlorsilan versetzt, und bei 60 °C für 16 Stunden in einem Trockenschrank inkubiert. Anschließend wurden die Gelstücke mittels Filtration über einen Büchner Trichter (Whatman® Filter, 125 mm, Grade 40) abgetrennt und in einem Vakuumtrockenschrank bei vermindertem Druck bis zur Gewichtskonstanz getrocknet (10 mbar, 120 °C), wodurch ein Aerogel erhalten wurde, welches anschließend mit den oben aufgeführten Methoden analysiert wurde.

Bestimmte analytische Daten des 1, Beispiels:

Dichte: 0,10 g/cm$^3$
BET: 587 m$^2$/g
C-Gehalt vor der Oberflächenmodifizierung: 9,2 Gew.-%
C-Gehalt nach der Oberflächenmodifizierung: 11,7 Gew.-%
TMS-Gruppen Belegung: 0,71 nm$^{-2}$

**Beispiel 2: Herstellung eines Aerogels aus Bindzil 17/750 und Kaliummethylsiliconat**

In einer verschließbaren Glasflasche wurden unter Rühren 56,6 g SiO$_2$-Nanosol zu einer auf 0°C gekühlten Lösung aus 4,8 g HCl Lösung (32 Gew.-%) und 34 g Wasser gegeben. Anschließend wurden unter Kühlung auf 0°C innerhalb 10 Minuten 7,6 g Kaliummethylsiliconat zugegeben, so dass sich ein pH Wert von 8 einstellte. Anschließend wurde die Probe auf RT erwärmt, wodurch die Gelbildung begann, welche innerhalb 45 Minuten abgeschlossen war. Das so erhaltene Lyogel, bei dem es sich in diesem Fall um ein Hydrogel handelte, wurde zur Alterung für 48 h bei 60°C inkubiert, anschließend wie in Beispiel 1 in Stücke kleiner 5 mm zerkleinert und die Gesamtheit der Gelstücke in zwei Teile aufgeteilt.
Ein Teil (ca. 50 g) der Gelstücke wurde zur Bestimmung des C-Gehalts vor der Oberflächenmodifizierung mit Wasser gewaschen und der Feststoff bei 120 °C und 10 mbar bis zur Gewichtskonstanz getrocknet und anschließend wie oben beschrieben analysiert.
Der zweite Teil (ca. 50 g) der Gelstücke wurde mit 100 ml Hexamethyldisiloxan überschichtet. Durch Zugabe von 10 g HCl (32%) und 10 g Ethanol als Phasenvermittler wurde das Hydrogel für 16 h bei 80°C silyliert, wobei die wässrige Phase aus den Poren verdrängt wurde. Die wässrige Phase wurde abgetrennt und das erhaltene, hydrophobe Lyogel über einen Büchner Trichter (Whatman® Filter, 125 mm, Grade 40) abfiltriert und bei 120 °C und 10 mbar bis zur Gewichtskonstanz getrocknet, wodurch ein Aerogel erhalten wurde, welches anschließend mit den oben aufgeführten Methoden analysiert wurde.

Bestimmte analytische Daten des 2. Beispiels:

Dichte: 0,11 g/cm$^3$
BET: 300 m$^2$/g
C-Gehalt vor der Oberflächenmodifizierung: 3,6 Gew.-%
C-Gehalt nach der Oberflächenmodifizierung: 3,8 Gew.-%
TMS-Gruppen Belegung: 0,11 nm$^{-2}$

**Vergleichsbeispiel 1: Gelbildung aus reinem SiO$_2$** (in Anlehnung an EP 0 948 395 B1)

In einem Becherglas wurden 150 g Wasser und 150 g Wasserglas gemischt und in einem Eisbad auf 10 °C gekühlt.
In einer Schraubflasche wurden 200 g Salzsäure (7,5 Gew.-%) vorgelegt, in einem Eisbad auf unter 10 °C gekühlt und mit einem Magnetrührer bei 500 UpM gerührt.
Die gekühlte Wasserglas-Lösung wurde über einen Tropftrichter langsam unter Rühren zu der Salzsäurelösung gegeben. Bei der Dosierung wurde darauf geachtet, dass die Temperatur nicht über 10 °C steigt. Bei einem pH-Wert von 5,2 wurde die Zugabe gestoppt und das Reaktionsgemisch auf Raumtemperatur erwärmt, wodurch Gelbildung stattfand. Für die Alterung wurde das erhaltene Lyogel in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten. Zur Entfernung der Salze wurden die Gelstücke fünfmal für jeweils 24 Stunden in 60 °C heißem, schwach alkalischem Wasser inkubiert (300 ml Wasser je 100 g Gel). Das Wasser wurde hierfür mit NaOH auf einen pH-Wert von 8,5 eingestellt. Das Wasser wurde dabei nach jeweils 24 Stunden durch Dekantieren abgetrennt und anschließend durch frisches, schwach alkalisches Wasser ersetzt.

10 g des erhaltenen, feuchten Gels wurden zur Bestimmung des C-Gehaltes vor der Oberflächenmodifizierung bei 180 °C in einem Trockenschrank bis zur Gewichtskonstanz getrocknet und anschließend wie oben beschrieben analysiert.

100 g des erhaltenen, feuchten Gels wurden vor der Oberflächenmodifizierung mit 200 ml einer Ethanol-Wasser-Mischung (50 Gew.-% Ethanol) überschichtet und für 16 Stunden bei Raumtemperatur in einem geschlossenen Gefäß inkubiert. Anschließend wurde das Gel mittels Filtration über einen Büchner Trichter (Whatman® Filter, 125 mm, Grade 40) abgetrennt. Für die Oberflächenmodifizierung wurden die erhaltenen Gelstücke in einer verschlossenen Schraubflasche mit 200 ml Hexamethyldisiloxan und 10,0 g Trimethylchlorsilan versetzt, geschüttelt und bei 60 °C für 16 Stunden in einem Trockenschrank inkubiert. Anschließend wurden die Gelstücke mittels Filtration über einen Büchner Trichter (Whatman® Filter, 125 mm, Grade 40) abgetrennt und in einem Vakuumtrockenschrank bei vermindertem Druck bis zur Gewichtskonstanz getrocknet (10 mbar, 120 °C), wodurch ein Aerogel erhalten wurde, welches anschließend mit den angegebenen Methoden analysiert wurde.

Bestimmte analytische Daten des 1. Vergleichsbeispiels:

Dichte: 0,20 g/cm$^3$
BET: 521 m$^2$/g
C-Gehalt vor der Oberflächenmodifizierung: < 0,1 Gew.-%
C-Gehalt nach der Oberflächenmodifizierung: 8,4 Gew.-%
TMS-Gruppen Belegung: 2,70 nm$^{-2}$

**Vergleichsbeispiel 2: Gelbildung aus Wasserglas und Kaliummethylsiliconat (durch Cokondensation)**

In einem Becherglas wurden 150,0 g Wasser, 75,0 g Wasserglas und 75,0 g Kaliummethylsiliconat gemischt und in einem Eisbad auf 10 °C gekühlt.

In einer Schraubflasche wurden 200 g Salzsäure (7,5 Gew.-%) vorgelegt, in einem Eisbad auf unter 10 °C gekühlt und mit einem Magnetrührer bei 500 UpM gerührt.

Die gekühlte Wasserglas-Kaliummethylsiliconat-Lösung wurde über einen Tropftrichter langsam unter Rühren zu der Salzsäurelösung gegeben. Bei der Dosierung wurde darauf geachtet, dass die Temperatur nicht über 10 °C steigt. Bei einem pH-Wert von 5,3 wurde die Zugabe gestoppt und das Reaktionsgemisch auf Raumtemperatur erwärmt, wodurch Gelbildung stattfand. Für die Alterung wurde das erhaltene Gel in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten. Zur Entfernung der Salze wurden die Gelstücke fünfmal für jeweils 24 Stunden in 60 °C heißem, schwach alkalischem Wasser inkubiert (300 ml Wasser je 100 g Gel). Das Wasser wurde hierfür mit NaOH auf einen pH-Wert von 8,5 eingestellt. Das Wasser wurde dabei nach jeweils 24 Stunden durch Dekantieren abgetrennt und anschließend durch frisches, schwach alkalisches Wasser ersetzt.

10 g des erhaltenen, feuchten Gels wurden zur Bestimmung des C-Gehaltes vor der Oberflächenmodifizierung bei 180 °C in einem Trockenschrank bis zur Gewichtskonstanz getrocknet und anschließend wie oben beschrieben analysiert.

100 g des erhaltenen, feuchten Gels wurden vor der Oberflächenmodifizierung mit 200 ml einer Ethanol-Wasser-Mischung (50 Gew.-% Ethanol) überschichtet und für 16 Stunden bei Raumtemperatur in einem geschlossenen Gefäß inkubiert. Anschließend wurde das Gel mittels Filtration über einen Büchner Trichter (Whatman® Filter, 125 mm, Grade 40) abgetrennt. Für die Oberflächenmodifizierung wurden die erhaltenen Gelstücke in einer verschlossenen Schraubflasche mit 200 ml Hexamethyldisiloxan und 10,0 g Trimethylchlorsilan versetzt, geschüttelt und bei 60 °C für 16 Stunden in einem Trockenschrank inkubiert. Anschließend wurden die Gelstücke mittels Filtration über einen Büchner Trichter (Whatman® Filter, 125 mm, Grade 40) abgetrennt und in einem Vakuumtrockenschrank bei vermindertem Druck bis zur Gewichtskonstanz getrocknet (10 mbar, 120 °C), wodurch ein Aerogel erhalten wurde, welches anschließend mit den angegebenen Methoden analysiert wurde.

Bestimmte analytische Daten des 2. Vergleichsbeispiels:

Dichte: 0,11 g/cm$^3$
BET: 644 m$^2$/g
C-Gehalt vor der Oberflächenmodifizierung: 8,7 Gew.-%
C-Gehalt nach der Oberflächenmodifizierung: 14,8 Gew.-%
TMS-Gruppen Belegung: 1,57 nm$^{-2}$

**Vergleichsbeispiel 3: Gelbildung aus [(CH$_3$)SiO$_{3/2}$]** (in Anlehnung an Shan Yun et. al., RSC Adv., 2014, 4, 4535-4542)

In einer Schraubflasche wurden 315 g Wasser und 3,0 g Cetyltrimethylammoniumbromid (Sigma-Aldrich) vorgelegt, unter Rühren (Magnetrührer, 500 UpM) mit 81,8 g Methyltrimethoxysilan versetzt und für 20 Minuten bei Raumtemperatur gerührt. Anschließend wurden unter Rühren 3,0 ml einer Ammoniaklösung (1,0 M) zugegeben, eine Minute nachgerührt und der Rührer entfernt, woraufhin die Gelbildung einsetzt. Für die Alterung wurde das erhaltene Gel in einem geschlossenen Gefäß für 16 Stunden bei 60 °C in einem Trockenschrank inkubiert.

Danach wurde das Gel durch ein Sieb mit einer Maschenweite von 5 mm gedrückt, um Stücke kleiner 5 mm zu erhalten. Zur Entfernung des eingesetzten Cetyltrimethylammoniumbromids und des Porenwassers wurden die Gelstücke zunächst dreimal für jeweils 24 Stunden in auf 50 °C erhitztem Ethanol (300 ml Ethanol je 100 g Gel) in einem geschlossen Gefäß bei 50 °C inkubiert. Das Ethanol wurde dabei nach jeweils 24 Stunden durch Dekantieren abgetrennt und anschließend durch frischen, auf 50 °C erhitzten Ethanol ersetzt. Anschließend wurden die Gelstücke dreimal für jeweils 24 Stunden in auf 50 °C erhitztem n-Hexan (300 ml n-Hexan je 100 g Gel) in einem geschlossen Gefäß inkubiert. Das n-Hexan wurde dabei nach jeweils 24 Stunden durch Dekantieren abgetrennt und anschließend durch frisches, auf 50 °C erhitztes n-Hexan ersetzt. Anschließend wurden die Gelstücke mittels Filtration über einen Büchner Trichter (Whatman® Filter, 125 mm, Grade 40) abgetrennt.

10 g des erhaltenen Gels wurden zur Bestimmung des C-Gehaltes vor der Oberflächenmodifizierung in einem Vakuumtrockenschrank bei vermindertem Druck bis zur Gewichtskonstanz getrocknet (10 mbar, 120 °C) und anschließend wie oben beschrieben analysiert.

50,0 g der erhaltenen Gelstücke wurden für die Oberflächenmodifizierung in einer verschlossenen Schraubflasche mit 250 ml n-Hexan und 5,0 g Trimethylchlorsilan versetzt, geschüttelt und bei 50 °C für 24 Stunden in einem Trockenschrank inkubiert. Anschließend wurden die Gelstücke mittels Filtration über einen Büchner Trichter (Whatman® Filter, 125 mm, Grade 40) abgetrennt, zweimal mit je 250 ml n-Hexan gewaschen und in einem Vakuumtrockenschrank bei vermindertem Druck bis zur Gewichtskonstanz getrocknet (10 mbar, 120 °C), wodurch ein Aerogel erhalten wurde, welches anschließend mit den angegebenen Methoden analysiert wurde.

Bestimmte analytische Daten des 3. Vergleichsbeispiels:

Dichte: 0,25 g/cm$^3$
BET: 615 m$^2$/g
C-Gehalt vor der Oberflächenmodifizierung: 18,0 Gew.-%
C-Gehalt nach der Oberflächenmodifizierung: 18,8 Gew.-%
TMS-Gruppen Belegung: 0,22 nm$^{-2}$

**Beispiel 3: Herstellung eines Aerogels aus Bindzil 17/750, Methyltriethoxysilan und Dimethyldiethoxysilan**

In einer verschließbaren Glasflasche wurden unter Rühren 120 g SiO$_2$-Nanosol zu einer auf 0°C gekühlten Lösung aus 3,2 g HCl Lösung (32 Gew.-%) und 22,6 g Wasser gegeben, wobei sich ein pH-Wert von 2,5 einstellte. Anschließend wurde unter Kühlung auf 0°C innerhalb 10 Minuten eine Mischung aus 15,2 g Methyltriethoxysilan und 6,3 g Dimethyldiethoxysilan zugegeben und für ca. 30 min gerührt. Anschließend wurde mit einer Ammoniaklösung (1 M) ein pH Wert von 8 eingestellt. Anschließend wurde die Probe auf RT erwärmt, wodurch die Gelbildung begann, welche innerhalb 45 Minuten abgeschlossen war. Das so erhaltene Lyogel wurde zur Alterung für 48 h bei 60°C inkubiert, anschließend wie in Beispiel 1 in Stücke kleiner 5 mm zerkleinert und die Gesamtheit der Gelstücke in zwei Teile aufgeteilt.

Ein Teil (ca. 50 g) der Gelstücke wurde zur Bestimmung des C-Gehalts vor der Oberflächenmodifizierung mit Wasser gewaschen, der Feststoff bei 120 °C und 10 mbar bis zur Gewichtskonstanz getrocknet und anschließend wie oben beschrieben analysiert.

Der zweite Teil (ca. 50 g) der Gelstücke wurde mit 100 ml Hexamethyldisiloxan überschichtet. Durch Zugabe von 10 g HCl (32%) und 10 g Ethanol als Phasenvermittler wurde das Hydrogel für 16 h bei 70°C silyliert, wobei die wässrige Phase aus den Poren verdrängt wurde. Die wässrige Phase wurde abgetrennt und das erhaltene, hydrophobe Lyogel über einen Büchner Trichter (Whatman® Filter, 125 mm, Grade 40) abfiltriert und bei 120 °C und 10 mbar bis zur Gewichtskonstanz getrocknet, wodurch ein Aerogel erhalten wurde, welches anschließend mit den oben aufgeführten Methoden analysiert wurde.

Bestimmte analytische Daten des 3. Beispiels:

Dichte: 0,16 g/cm$^3$
BET: 360 m$^2$/g
C-Gehalt vor der Oberflächenmodifizierung: 7,7 Gew.-%
C-Gehalt nach der Oberflächenmodifizierung: 9,2 Gew.-%
TMS-Gruppen Belegung: 0,69 nm$^{-2}$

Tabelle 1: Übersicht der bestimmten analytischen Daten aller Beispiele

| Beispiel | Dichte [g/cm$^3$] | $C_{ohne\ TMS}$ [Gew.-%] | $C_{mit\ TMS}$ [Gew.-%] | $\Delta C$ [Gew.-%] | BET [m$^2$/g] | TMS-Gruppen Belegungsgrad [nm$^{-2}$] |
|---|---|---|---|---|---|---|
| 1 | 0,10 | 9,2 | 11,7 | 2,5 | 587 | 0,71 |
| 2 | 0,11 | 3,6 | 3,8 | 0,2 | 300 | 0,11 |
| 3 | 0,16 | 7,7 | 9,2 | 1,5 | 360 | 0,69 |
| Vergleich 1 | 0,20 | < 0,1 | 8,4 | 8,4 | 521 | 2,70 |
| Vergleich 2 | 0,11 | 8,7 | 14,8 | 6,1 | 644 | 1,57 |
| Vergleich 3 | 0,25 | 18,0 | 18,8 | 0,8 | 615 | 0,22 |

**Patentansprüche**

1. Gele ausgewählt aus Lyo- oder Aerogelen enthaltend Primärpartikel, die aus oxidischen Einheiten und [R$_x$-SiO$_{(4-x)/2}$]-Einheiten aufgebaut sind, wobei die Primärpartikel eine Änderung der Konzentration an [R$_x$SiO$_{(4-x)/2}$]-Einheiten von innen nach außen aufweisen,
wobei x gleich oder verschieden sein kann und 1 oder 2 ist und
R gleich oder verschieden sein kann und Wasserstoff oder ein organischer, substituierter oder unsubstituierter Rest ist
und wobei die oxidischen Einheiten [SiO$_{4/2}$]-Einheiten enthalten.

2. Gele nach Anspruch 1, **dadurch gekennzeichnet, dass** die Primärpartikel eine Zunahme der Konzentration an [R$_x$SiO$_{(4-x)/2}$]-Einheiten von innen nach außen aufweisen.

3. Gele nach einem oder mehreren der Ansprüche 1 oder 2,
**dadurch gekennzeichnet, dass** die Primärpartikel in Form eines Kern-Schale-Modells aufgebaut sind, wobei der Kern eine Konzentration an [R$_x$SiO$_{(4-x)/2}$]-Einheiten von unter 20 mol-% enthält und die Schale eine Konzentration an [R$_x$SiO$_{(4-x)/2}$]-Einheiten von über 80 mol-% enthält.

4. Gele nach einem oder mehreren der Ansprüche 1 bis 3,
**dadurch gekennzeichnet, dass** der Rest R eine Methyl-Gruppe ist.

5. Gele nach einem oder mehreren der Ansprüche 1 bis 4,
**dadurch gekennzeichnet, dass** der Belegungsgrad an monofunktionellen Einheiten an der Oberfläche unter 1,5 Gruppen pro nm$^2$ liegt.

6. Gele nach einem oder mehreren der Ansprüche 1 bis 5,

**dadurch gekennzeichnet, dass** es sich um Aerogele mit einer Dichte unter 0,25 g/cm$^3$ handelt.

7.  Gele nach einem oder mehreren der Ansprüche 1 bis 6,
    **dadurch gekennzeichnet, dass** es sich um Aerogele mit einem Kohlenstoffgehalt unter 15 Gew.-% handelt.

8.  Gele nach einem oder mehreren der Ansprüche 1 bis 7,
    **dadurch gekennzeichnet, dass** es sich um Aerogele mit einer Dichte unter 0,15 g/cm$^3$, einem Belegungsgrad an monofunktionellen Einheiten von höchstens 1 pro nm$^2$, einem Kohlenstoffgehalt von höchstes 8 Gew.-% und einer BET-Oberfläche größer 300 m$^2$/g handelt.

9.  Gele nach einem oder mehreren der Ansprüche 1 bis 8,
    **dadurch gekennzeichnet, dass** als oxidische Einheiten nur [SiO$_{4/2}$]-Einheiten vorliegen.

10. Verfahren zur Herstellung von Gelen entsprechend einem oder mehreren der Ansprüche 1 bis 9 indem,

    i) ein Sol enthaltend kolloidale Partikel, wobei die kolloidalen Partikel oxidische Einheiten enthalten, vorgelegt wird,
    wobei die oxidischen Einheiten [SiO$_{4/2}$]-Einheiten enthalten
    und wobei die Sole durch Neutralisation von stark basischen Alkalisilikaten oder durch Hydrolyse von Alkoxysilanen hergestellt werden,
    ii) die Partikel im Sol mit [R$_x$SiO$_{(4-x)/2}$]-Einheiten zur Reaktion gebracht werden,
    wobei x gleich oder verschieden sein kann und 1 oder 2 ist und
    R gleich oder verschieden sein kann und Wasserstoff oder ein organischer, substituierter oder unsubstituierter Rest ist
    iii) und aus dem Sol ein Gel gebildet wird.

11. Verfahren nach Anspruch 10 **dadurch gekennzeichnet, dass** als Ausgangsstoff Lösungen von kolloidalen [SiO$_{4/2}$]-Einheiten enthaltenden Partikeln eingesetzt werden, wobei die Partikel einen mittleren Durchmesser im Bereich von 1 bis 8 nm haben.

12. Verfahren nach einem oder mehreren der Ansprüche 10 oder 11, **dadurch gekennzeichnet, dass** das Gel durch Oberflächenmodifizierung mit monofunktionellen Einheiten belegt wird.

13. Verfahren nach einem oder mehreren der Ansprüche 10 bis 12, **dadurch gekennzeichnet, dass** das Gel getrocknet wird.

14. Verwendung der Gele nach einem oder mehreren der Ansprüche 1 bis 9 oder hergestellt nach einem Verfahren entsprechend einem oder mehreren der Ansprüche 10 bis 13, wobei es sich bei den Gelen um Aerogele handelt, in Anwendungen zur thermischen und/oder akustischen Isolierung.

15. Verwendung der Gele nach einem oder mehreren der Ansprüche 1 bis 9 oder hergestellt nach einem Verfahren entsprechend einem oder mehreren der Ansprüche 10 bis 13 in kosmetischen, medizinischen oder für chromatographische Anwendungen, sowie als Katalysatoren oder Katalysatorträger.

**Claims**

1.  Gels selected from among lyogel- or aerogel-containing primary particles which are made up of oxidic units and [R$_x$SiO$_{(4-x)/2}$] units, wherein the primary particles have a change in the concentration of [R$_x$SiO$_{(4-x)/2}$] units from the inside to the outside,
    where the indices x can be identical or different and are in each case 1 or 2 and
    the radicals R can be identical or different and are each hydrogen or an organic, substituted or unsubstituted radical, and the oxidic units contain [SiO$_{4/2}$] units.

2.  Gels according to Claim 1, **characterized in that** the primary particles have an increase in the concentration of [R$_x$SiO$_{(4-x)/2}$] units from the inside to the outside.

3.  Gels according to one or more of Claims 1 and 2, **characterized in that** the primary particles are built up in the form of a core-shell model, where the core contains a concentration of $[R_xSiO_{(4-x)/2}]$ units of less than 20 mol% and the shell contains a concentration of $[R_xSiO_{(4-x)/2}]$ units of more than 80 mol%.

4. Gels according to one or more of Claims 1 to 3, **characterized in that** the radical R is a methyl group.

5. Gels according to one or more of Claims 1 to 4, **characterized in that** the degree of coverage with monofunctional units on the surface is less than 1.5 groups per $nm^2$.

6. Gels according to one or more of Claims 1 to 5, **characterized in that** it is an aerogel having a density of less than 0.25 $g/cm^3$.

7. Gels according to one or more of Claims 1 to 6, **characterized in that** it is an aerogel having a carbon content of less than 15% by weight.

8. Gels according to one or more of Claims 1 to 7, **characterized in that** it is an aerogel having a density of less than 0.15 $g/cm^3$, a degree of coverage with monofunctional units of not more than 1 per $nm^2$, a carbon content of not more than 8% by weight and a BET surface area of greater than 300 $m^2/g$.

9. Gels according to one or more of Claims 1 to 8, **characterized in that** only $[SiO_{4/2}]$ units are present as oxidic units.

10. Process for producing gels corresponding to one or more of Claims 1 to 9 by

   i) placing a sol containing colloidal particles, where the colloidal particles contain oxidic units, in a reaction vessel, where the oxidic units contain $[SiO_{4/2}]$ units and the sols are produced by neutralization of strongly basic alkali metal silicates or by hydrolysis of alkoxysilanes,
   ii) reacting the particles in the sol with $[R_xSiO_{(4-x)/2}]$ units,
   where the indices x can be identical or different and are in each case 1 or 2 and
   the radicals R can be identical or different and are each hydrogen or an organic, substituted or unsubstituted radical,
   iii) and forming a gel from the sol.

11. Process according to Claim 10, **characterized in that** solutions of colloidal particles containing $[SiO_{4/2}]$ units are used as starting material, where the particles have an average diameter in the range from 1 to 8 nm.

12. Process according to one or more of Claims 10 and 11, **characterized in that** the gel is covered with monofunctional units by surface modification.

13. Process according to one or more of Claims 10 to 12, **characterized in that** the gel is dried.

14. Use of the gels according to one or more of Claims 1 to 9 or produced by a process corresponding to one or more of Claims 10 to 13, wherein the gels are aerogels, in applications for thermal and/or acoustic insulation.

15. Use of the gels according to one or more of Claims 1 to 9 or produced by a process corresponding to one or more of Claims 10 to 13 in cosmetic, medical or chromatographic applications or as catalysts or catalyst supports.

**Revendications**

1. Gels choisis parmi les lyo- et aérogels contenant des particules primaires, qui sont formées par des unités oxydiques et des unités $[R_xSiO_{(4-x)/2}]$, les particules primaires présentent une modification de la concentration en unités $[R_x-SiO_{(4-x)/2}]$ de l'intérieur vers l'extérieur, les x pouvant être identiques ou différents, et valant 1 ou 2, et les R pouvant être identiques ou différents, et représentant l'hydrogène ou un radical organique substitué ou non substitué,
et les unités oxydiques contenant des unités $[SiO_{4/2}]$.

2. Gels selon la revendication 1, **caractérisés en ce que** les particules primaires présentent une augmentation de la concentration en unités $[R_xSiO_{(4-x)/2}]$ de l'intérieur vers l'extérieur.

**3.** Gels selon une ou plusieurs des revendications 1 ou 2, **caractérisés en ce que** les particules primaires sont configurées sous la forme d'un modèle noyau-enveloppe, le noyau contenant une concentration d'unités $[R_xSiO_{(4-x)/2}]$ inférieure à 20 % en moles et l'enveloppe contenant une concentration d'unités $[R_xSiO_{(4-x)/2}]$ supérieure à 80 % en moles.

**4.** Gels selon une ou plusieurs des revendications 1 à 3, **caractérisés en ce que** le radical R est un groupe méthyle.

**5.** Gels selon une ou plusieurs des revendications 1 à 4, **caractérisés en ce que** le degré de recouvrement de la surface en unités monofonctionnelles est inférieur à 1,5 groupe par $nm^2$.

**6.** Gels selon une ou plusieurs des revendications 1 à 5, **caractérisés en ce qu'**il s'agit d'aérogels ayant une densité inférieure à 0,25 $g/cm^3$.

**7.** Gels selon une ou plusieurs des revendications 1 à 6, **caractérisés en ce qu'**il s'agit d'aérogels ayant une teneur en carbone inférieure à 15 % en poids.

**8.** Gels selon une ou plusieurs des revendications 1 à 7, **caractérisés en ce qu'**il s'agit d'aérogels ayant une densité inférieure à 0,15 $g/cm^3$, un degré de recouvrement en unités monofonctionnelles d'au plus 1 par $nm^2$, une teneur en carbone d'au plus 8 % en poids et une surface BET supérieure à 300 $m^2/g$.

**9.** Gels selon une ou plusieurs des revendications 1 à 8, **caractérisés en ce que** seules des unités $[SiO_{4/2}]$ sont présentes en tant qu'unités oxydiques.

**10.** Procédé de fabrication de gels selon une ou plusieurs des revendications 1 à 9, selon lequel

i) un sol contenant des particules colloïdales, les particules colloïdales contenant des unités oxydiques, est chargé initialement,
les unités oxydiques contenant des unités $[SiO_{4/2}]$,
et les sols étant fabriqués par neutralisation de silicates alcalins fortement basiques ou par hydrolyse d'alcoxysilanes,
ii) les particules dans le sol sont mises en réaction avec des unités $[R_xSiO_{(4-x)/2}]$,
les x pouvant être identiques ou différents, et valant 1 ou 2, et
les R pouvant être identiques ou différents, et représentant l'hydrogène ou un radical organique substitué ou non substitué,
iii) et un gel est formé à partir du sol.

**11.** Procédé selon la revendication 10, **caractérisé en ce que** des solutions de particules contenant des unités $[SiO_{4/2}]$ colloïdales sont utilisées en tant que matière première, les particules ayant un diamètre moyen dans la plage allant de 1 à 8 nm.

**12.** Procédé selon une ou plusieurs des revendications 10 ou 11, **caractérisé en ce que** le gel est recouvert avec des unités monofonctionnelles par modification de surface.

**13.** Procédé selon une ou plusieurs des revendications 10 à 12, **caractérisé en ce que** le gel est séché.

**14.** Utilisation des gels selon une ou plusieurs des revendications 1 à 9 ou fabriqués par un procédé selon une ou plusieurs des revendications 10 à 13, les gels étant des aérogels, dans des applications pour l'isolation thermique et/ou acoustique.

**15.** Utilisation des gels selon une ou plusieurs des revendications 1 à 9 ou fabriqués par un procédé selon une ou plusieurs des revendications 10 à 13 dans des applications cosmétiques, médicales ou chromatographiques, ainsi qu'en tant que catalyseurs ou supports de catalyseurs.

Fig. 1:

Fig. 2:

Fig. 3:

Fig. 4:

A

B

C

**IN DER BESCHREIBUNG AUFGEFÜHRTE DOKUMENTE**

**In der Beschreibung aufgeführte Patentdokumente**

- EP 0948395 B1
- EP 0948395 A

**In der Beschreibung aufgeführte Nicht-Patentliteratur**

- Aerogels Handbook Series: Advances in Sol-Gel Derived Materials and Technologies. Springer Verlag, 2011
- **AEGERTER et al.** Aerogels Handbook. 2011
- **EINLEITUNG A. V. RAO et al.** *J. Colloid Interface Sci.,* 2006, vol. 300, 279-285
- **A. V. RAO et al.** *Appl. Surf. Sci.,* 2007, vol. 253, 6032-6040
- **K. KANAMORI et al.** *Adv. Mater.,* 2007, vol. 19, 1589-1593